# EUROPEAN PATENT SPECIFICATION

(11) **EP 3 983 565 B1**
(45) Date of publication and mention of the grant of the patent: **04.10.2023**
(21) Application number: 20731494.9
(22) Date of filing: 12.06.2020
(51) Int. Cl.: C12Q 1/6886, C12Q 1/6879

(54) **METHOD OF STRATIFYING SUBJECTS INTO SUB-GROUPS FOR THERAPEUTIC TREATMENT**
VERFAHREN ZUM STRATIFIZIERUNG VON PROBANDEN IN UNTERGRUPPEN ZUR THERAPEUTISCHEN BEHANDLUNG
PROCÉDÉ DE STRATIFICATION DE SUJETS EN SOUS-GROUPES POUR UN TRAITEMENT THÉRAPEUTIQUE

(30) Priority: 14.06.2019 GB 201908565
(43) Date of publication of application: 20.04.2022
(73) Proprietor: Cray Innovation AB, 752 37 Uppsala (SE)
(72) Inventor: FORSBERG, Lars, 756 51 Uppsala (SE); DUMANSKI, Jan, 756 53 Uppsala (SE)
(74) Representative: Dehns
(86) International application number: PCT/EP2020/066372
(87) International publication number: WO 2020/249774

(56) References cited:
- WO-A1-2015/052190
- WO-A1-2018/039567
- WO-A2-2007/140958
- LARS A FORSBERG ET AL: "Mosaic loss of chromosome Y in peripheral blood is associated with shorter survival and higher risk of cancer", NATURE GENETICS, vol. 46, no. 6, 28 April 2014 (2014-04-28) , pages 624-628, XP055170832, ISSN: 1061-4036, DOI: 10.1038/ng.2966
- DUMANSKI JAN P ET AL: "Mosaic Loss of Chromosome Y in Blood Is Associated with Alzheimer Disease", AMERICAN JOURNAL OF HUMAN GENETICS, AMERICAN SOCIETY OF HUMAN GENETICS, CHICAGO, IL, US, vol. 98, no. 6, 23 May 2016 (2016-05-23), pages 1208-1219, XP029567322, ISSN: 0002-9297, DOI: 10.1016/J.AJHG.2016.05.014
- PREDRAG NOVESKI ET AL: "Loss of Y Chromosome in Peripheral Blood of Colorectal and Prostate Cancer Patients", PLOS ONE, vol. 11, no. 1, 8 January 2016 (2016-01-08), page e0146264, XP055629420, DOI: 10.1371/journal.pone.0146264

## Description

### Field of the invention

The present invention relates to methods for stratifying male patients into sub-groups for treatment, e.g. differential treatment, with immunotherapeutic agents based on determining, in samples obtained from the patients, the fraction of nucleated blood cells (i.e. leucocytes) that have lost chromosome Y. We have shown that loss of chromosome Y (LOY) is associated with changes in expression of a wide number of autosomal genes that are important for normal immune cell functions. This underlies the present invention, and the invention also includes the use of such LOY-associated genes as biomarkers for responsiveness to immunotherapies.

### Background

Chromosome Y is required in mammals to override the development of the default female sex. The human X and Y chromosomes evolved from a pair of autosomes over the past 300 million years. During the early history of chromosome Y, a genetic decay with gene loss has occurred. However, the mammalian chromosome Y has been remarkably stable for the past 25 million years. Molecular studies of chromosome Y are often challenging due to its rich content of highly repetitive, low-level redundancy and palindromic sequences. Although the male DNA was included in the early genome sequencing projects, the Y chromosome was largely ignored, due to difficulties in sequence assembly. Nevertheless, chromosome Y contains genes important for a wide range of functions, suggesting that it is far from a gene desert. Despite this, the notion of chromosome Y being a genetic wasteland has been difficult to erase.

Since the earliest cytogenetic analyses, loss of chromosome Y (LOY) has been known to occur in hematopoietic cells, resulting in a complete lack of almost 2% of the haploid nuclear genome in a cell with this aneuploidy. However, the phenotypical consequences of LOY, on the level of a cell or an entire organism, have remained unclear. The prevailing consensus over several decades suggested that LOY should be considered phenotypically neutral and related to normal aging. This further added to the misconception surrounding chromosome Y as being important for male sex determination and other male-specific traits, but largely insignificant beyond that. Recent epidemiological analyses have challenged the view that LOY in blood cells is phenotypically neutral. Studies have identified increased risks for men with LOY in connection with all-cause mortality (Forsberg et al., 2014; Loftfield et al., 2018), Alzheimer's disease (Dumanski et al., 2016), various forms of cancer (Forsberg et al., 2014; Ganster et al., 2015; Loftfield et al., 2019; Noveski et al., 2016), autoimmune conditions (Lleo et al., 2013; Persani et al., 2012), age-related macular degeneration Grassmann et al., 2018), cardiovascular disease (Haitjema et al., 2017), type 2 diabetes and obesity (Loftfield et al., 2018). WO 2015/052190 and WO 2015/121317 respectively disclose methods for predicting risk of cancer, particularly non-haematological cancer, and Alzheimer's disease (AD) based on performing a determination of LOY in cells from samples from male subjects. Particularly, the determinations may be performed in leucocytes (nucleated blood cells).

LOY in blood is the most frequent post-zygotic mutation, detectable in 20% of the UK Biobank male population, reaching 40% in 80 year old males, and 57% in 93 year old males. Furthermore, LOY is not restricted to the hematopoietic system, since it has also been described in other non-cancerous tissues, although with much lower frequencies. Our understanding of why LOY occurs is also limited. Strong associations with age and smoking have been reported, and these factors are related to a continuously increasing mutational load throughout the genome in all somatic cells, eventually also affecting genes that are responsible for correct segregation of chromosomes. Inherited genetic predisposition for LOY has also been described.

Crucially, it is not known whether associations between LOY and increased risks for disease represent causal relationships, and if so, what the underlying mechanisms could be. In the work underlying this invention, we have been able to move from epidemiological associations to mechanistic explanations of the relationship between LOY and disease. As described further in the Examples below, we have further studied (i) LOY in patients with prostate cancer (PC) and AD, (ii) changes in transcriptomes of single cells and cellular subsets in bulk; and (iii) alterations of plasma protein levels in men with LOY. This has led to the development of the present invention.

WO 2007/140958 discloses methods for identifying patients who will respond positively to immunotherapy based on their gene expression profile, and discloses the use of CCL5 and CD3D as markers for an immunotherapy response. WO 2018/039567 discloses systems and methods for the prediction of the treatment outcome for immune therapy, and discloses the use of LAG3 as a marker for an immunotherapy response.

### Summary of the invention

We have shown that different diseases may be associated with LOY in particular specific fractions of leucocytes. Particularly, Alzheimer's disease and prostate cancer patients demonstrate more LOY in NK cells and CD4+ T lymphocytes, respectively. This has allowed us to refine the methods of WO 2015/052190 and WO 2015/121317 above, to examine LOY in specific leucocyte subsets as set out below.

Furthermore, gene expression was found to be profoundly altered in cells with LOY in a pleiotropic fashion and numerous autosomal genes have been identified showing LOY-associated transcriptional effect ("LATE"), that are important for normal immune cell functions. Proteomic analysis also indicated that LOY leaves a footprint in the plasma proteome. Thus, LOY in the leucocytes of a subject may impact on, or affect, the functioning of the immune system of that subject. It follows from these observations that LOY in the leucocytes of a male subject, and in particular in the immune cells of the subject, may affect an immune response of the subject and therefore the response of the subject to immunotherapy, or in other words to any therapy which affects or modulates the immune system. This has led to the proposal that LOY may be used to stratify subjects for immunotherapy, in order to take into account the effect, or the possible effect, of LOY on the response of the subject to the immunotherapy. This may apply particularly in the recruitment of patients to clinical trials, but may also be of utility in the clinic, in helping to assess potential treatments for a patient. Additionally, as indicated above, we have identified a number of specific genes (termed "LATE genes" herein) which are associated with the LOY, and which further may be assessedas biomarkers in a subject to stratify the subject for an immunotherapy.

Accordingly in a first aspect, the present disclosure, (and more particularly the present invention) provides a method of stratifying a male subject for immunotherapy, said method comprising:
(a) determining in a sample obtained from the male subject a fraction of leucocytes that have lost chromosome Y;
(b) comparing said fraction to a predefined threshold; and
(c) based on said comparison between said fraction and said predefined threshold, assigning the male subject to either a LOY+ or LOY- sub-group, and stratifying the subject for said immunotherapy based on said sub-group.

In other words, step (c) may comprise assigning the subject for said immunotherapy based on said sub-group (i.e. based on the LOY sub-group into which the subject is assigned). In an embodiment step (c) may comprise assigning the subject into a treatment group for the immunotherapy (i.e. into a treatment sub-group). The treatment groups (or sub-groups) may be assigned, or classified, on various bases. For example, based on the LOY sub-group classification, a subject may be assigned to a different therapy, or may be assigned to therapy or no therapy, or the subject may simply be considered or assessed in the course of the therapy taking his LOY status into account. For example, response to the immunotherapy may be assessed, and may be correlated to the LOY status of the subject. Thus, the different treatment groups (i.e. sub-groups) may be differentiated, based on the LOY determination. In an embodiment, step (c) may comprise determining the immunotherapy for the subject, and/or for the LOY sub-group, e.g. determining an appropriate treatment for the subject and/or each LOY sub-group. More particularly, the subject may be assigned to differential treatment (e.g. immunotherapy) based on the LOY-sub-group determination. In an embodiment, the method may therefore be for stratifying a male subject into a sub-group for differential treatment with an immunotherapeutic agent.

In an embodiment, the method may be used to stratify a group of male subjects into sub-groups for the immunotherapy, e.g. for differential treatment in the immunotherapy, wherein based on the LOY sub-group in which each subject is assigned, the subject is assigned to an immunotherapy sub-group.

In another aspect, the present disclosure and invention provides a method of identifying the likelihood of response of a male subject to an immunotherapy, said method comprising:
(a) determining in a sample from the subject the fraction of leucocytes that have lost chromosome Y; and
(b) correlating the fraction of leucocytes that have lost chromosome Y to the likelihood that the subject will respond to treatment with the immunotherapy.

More particularly, the method may involve predicting the response (or the likelihood of response) of the male subject to an immunotherapy.

Step (b) may comprise comparing the fraction of leucocytes that have lost chromosome Y (i.e. the fraction of leucocyte cells with LOY), to a control value (e.g. a threshold value). Thus, the method may comprise comparing the fraction of leucocytes that have lost chromosome Y to a predefined threshold. Based on such a comparison, the response of the subject to the immunotherapy may be predicted. For example, if the fraction of leucocytes with LOY is equal to or below the control value (e.g. threshold), the male subject may be predicted to respond, or may be identified as likely to respond to the immunotherapy. If the fraction of leucocytes is above the control (e.g. threshold) value, the male subject may be predicted not to respond, or may be identified as unlikely to respond to the immunotherapy. In an embodiment of the method of this aspect, based on the fraction of leucocytes determined to have LOY, and the comparison to a predefined threshold, the subject may be determined to be LOY+ or LOY-, and based on this classification, the response to immunotherapy may be predicted, or the likelihood of response may be identified. This is discussed further below.

As noted above, LOY in leucocytes of a subject may be associated with changes, more particularly with a disturbance or dysregulation, in the expression of various autosomal genes. The methods herein may thus further comprise a step of determining the expression of one or more such genes in a sample obtained from the subject. As will be described in more detail below, the expression of the genes may be determined at the nucleic acid level, e.g. at the level of mRNA, or at the protein level, and may be determined in a cell-containing sample of the subject (e.g. the level of expression may be determined in cells in the sample), or in cell-free sample such as plasma or serum, or any blood-derived sample. This step may comprise determining the level or amount of the mRNA or protein in the sample. The level of gene expression determined in this step may be compared to a control level, as discussed further below. The level of expression determined may further be used to stratify the subjects.

Table 4 lists 134 LATE genes having functional links to the immune system, cancer or AD. Table 2 lists 95 LATE genes of particular interest which are associated with the function of the immune system. The expression of any of the genes listed in any of these Tables may be assessed, or determined, according to this aspect (i.e. in a step of determining the expression of one or genes in a sample from the subject). Thus, one or more of the genes may be assessed, in any combination.

In any of the methods presented herein, the subject may be any mammal. In particular embodiments, the subject is a human.

### Description of Figures

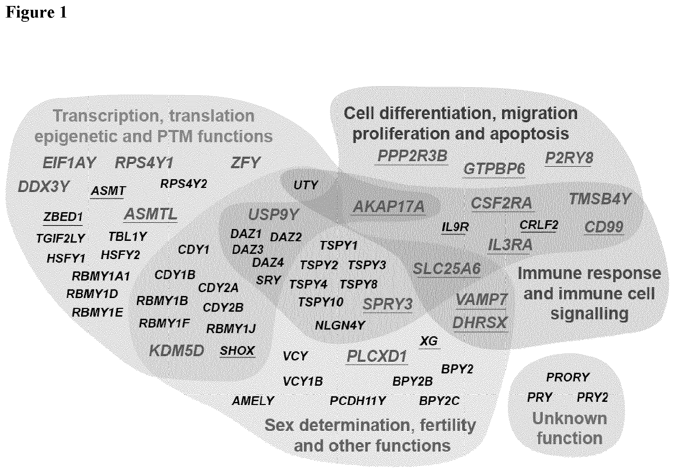

Figure 1 shows a summary of normal functions of the 64 protein coding genes on chromosome Y, 19 located in the pseudo-autosomal regions (PAR, underlined) and 45 in the male specific region of chromosome Y (MSY). Red color indicates 20 genes expressed in leukocytes studied by us using RNAseq, 13 in PAR and 7 in MSY. Gene ontology (GO) analyses were performed for each chromosome Y gene to identify known biological functions and the identified GO terms were annotated into four functional categories. The categories were: "*Transcription*, *translation epigenetic and post-translational modifications (PTM) functions*" (orange area), "*Cell differentiation, migration proliferation and apoptosis*" (grey area), *"Sex determination, fertility and other functions"* (blue area) and "*Immune response and immune cell signaling*" (violet area). Functions for three genes is unknown (green area).

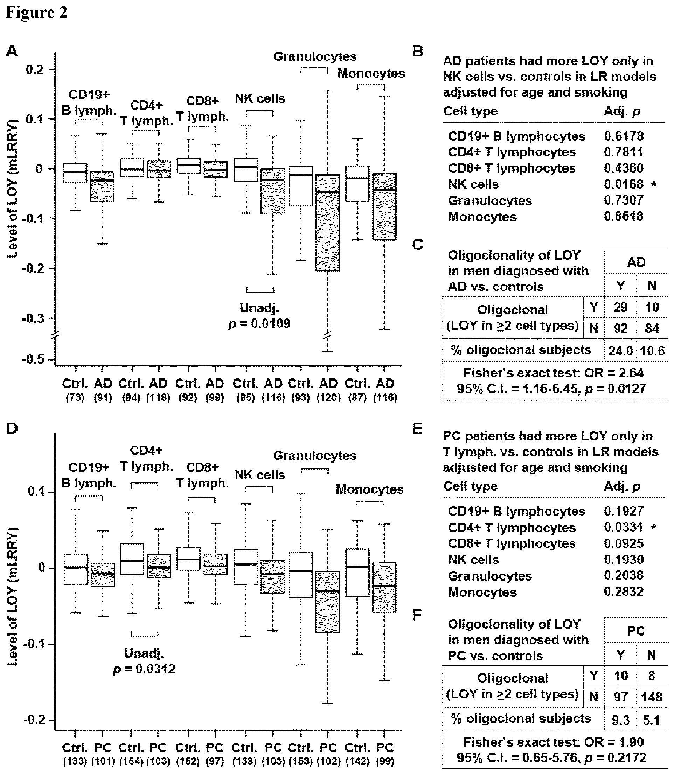

Figure 2 shows a comparison of the level of LOY in six populations of leukocytes in men diagnosed with Alzheimer's disease (AD) or prostate cancer (PC) vs controls. Leukocytes were sorted by FACS, followed by SNP-array genotyping of DNA from each cell fraction and calculation of the median Log R Ratio of the probes in the male specific part of chromosome Y (mLRRY). The numbers in parentheses under the X-axes in panels A and D denote the number of subjects studied for each cell type. Panels A and D show results from unadjusted analyses and panels B and E describe the results from adjusted logistic regression models. Panels C and F show results from investigation of LOY oligoclonality in men with AD and PC diagnoses vs controls. Abbreviations: Ctrl. = control, AD = Alzheimer's disease, PC = prostate cancer, LR = logistic regression, OR = odds ratio, lymph.= lymphocytes and NK = natural killer.

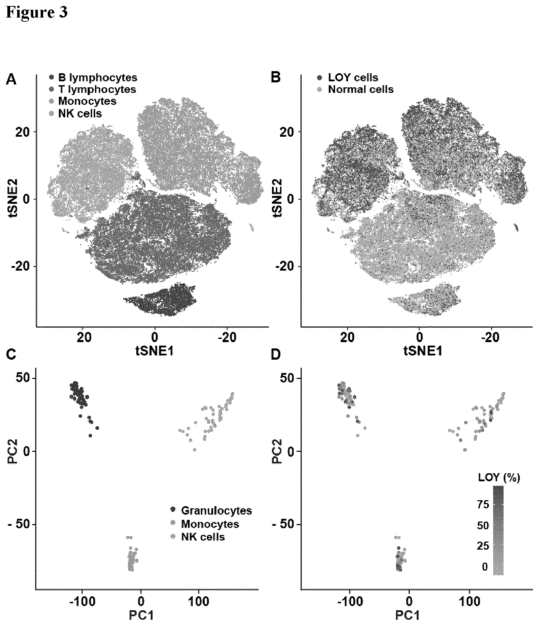

Figure 3 shows the distribution and level of loss of chromosome Y (LOY) in leukocytes from aging men using two RNA-sequencing platforms. Panels A and B show results from single cell RNA sequencing of peripheral blood mononuclear cells (PBMCs) collected from 29 men, 26 diagnosed with Alzheimer's disease. The tSNE plot in panel A shows pooled data from 73,606 PBMCs, each dot representing a single cell, in four cell types that are distinguished by colors. Panel B shows the distribution of cells displaying LOY in different cell types by coloring LOY cells in red and normal cells in grey. Panels C and D show results from bulk RNA sequencing (RNAseq) performed in three cell types sorted by FACS from 51 individuals. Panel C displays a principal component analysis based on global gene expression where each dot represents one cell type in one individual. The distance between dots indicate similarity in gene expression. Panel D shows the level of LOY mosaicism in each sample by a gradient of red and grey where red indicates a high level of LOY.

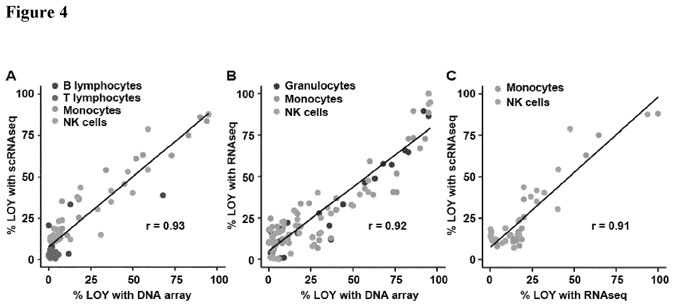

Figure 4 shows a comparison of performance of two RNA-sequencing platforms for estimation of the level of LOY in leukocytes. From the same set of blood samples, LOY analysis was performed in pairwise comparisons between scRNAseq vs array-based SNP genotyping (panel A), RNAseq vs array genotyping (panel B) and scRNAseq vs RNAseq (panel C). For the single cell data, pseudo-bulk samples were created for these comparisons by pooling the single cell data from each cell type for each individual. Abbreviations: scRNAseq = single cell RNA sequencing, RNAseq = bulk RNA sequencing, r = Pearson's correlation coefficient.

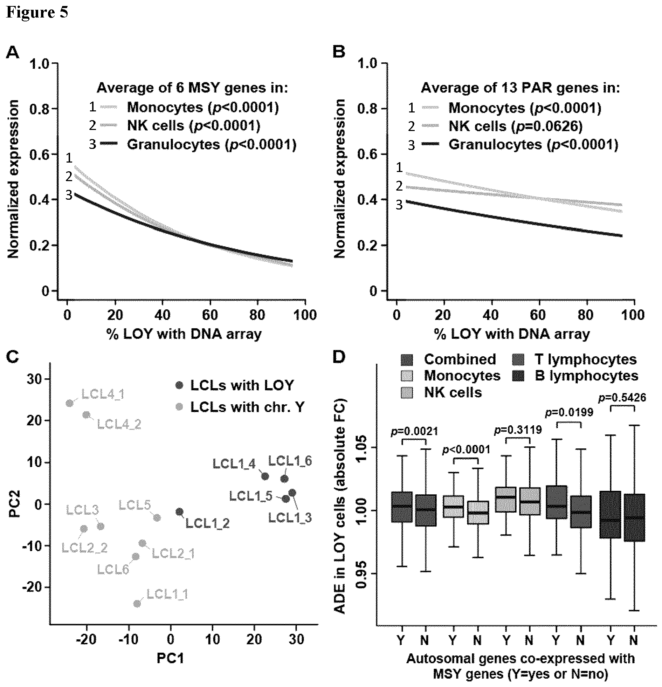

Figure 5 shows LOY Associated Transcriptional Effect (LATE) observed *in vivo* and *in vitro.* Panels A and B show decreased level of expression of 6 genes in the male-specific part of chromosome Y (MSY) (*RPS4Y1, ZFY, USP9Y, DDX3Y, KDM5D* and *EIF1AY*) and 12 genes in the pseudo autosomal regions of chromosomes X and Y (PAR) (*GTPBP6, PPP2R3B, CSF2RA, SLC25A6, ASMTL, P2RY8, AKAP17A, DHRSX, CD99, VAMP7, PLCXD1, IL3RA* and *SPRY3*), respectively. Gene expression was estimated using RNAseq in three FACS sorted cell types *in vivo.* Panel C shows a principal component analysis plot of global gene expression in lymphoblastoid cell lines (LCLs). LCLs with and without LOY are shown, and donor identity are specified by numbers (i.e. LCL2_1 and LCL2_2 are from donor 2). Distance between dots indicates similarity in global gene expression and analysis of the variance in PC1 shows that LCLs with LOY have altered global autosomal expression compared to normal LCLs (Kolmogorov-Smirnov test: D=1.0, *p*=0.0016). Panel D shows results from co-expression analysis and autosomal differential expression (ADE) in single cells with LOY. Autosomal genes that are normally co-expressed with MSY genes displayed a higher level of differential expression in single cells with LOY compared with the control genes without normal co-expression with MSY genes (Wilcoxon rank sum test: *p*=0.0021).

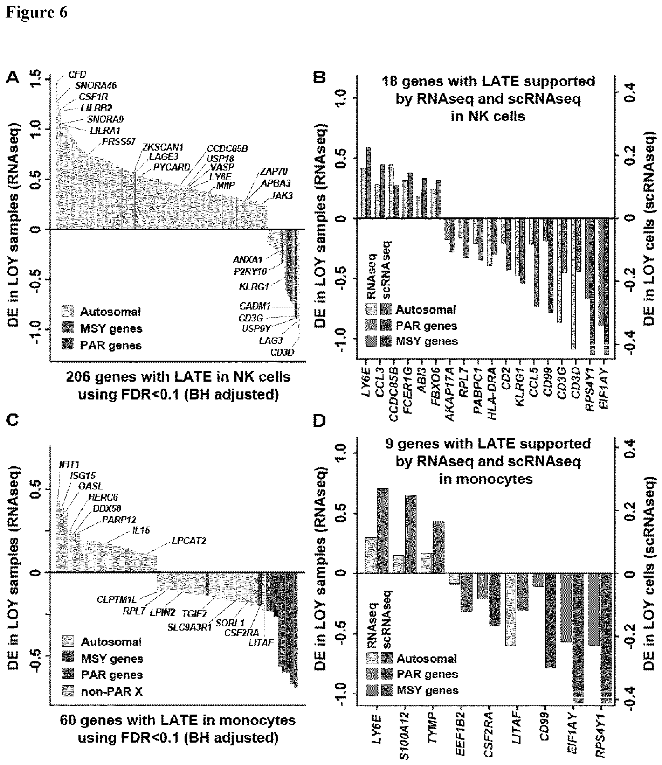

Figure 6 shows the level of differential expression (DE) of genes showing substantial LATE in NK cells and monocytes *in vivo.* Panel A shows the level of DE in 206 genes with significant dysregulation after correction for multiple testing (FDR<0.1) and with at least an average of 100 reads per gene in RNAseq data from NK cells. Names are shown for LATE genes involved in immune system functions and/or cancer and/or development of Alzheimer's disease. Panel B shows DE in 18 genes detected with both RNAseq and scRNAseq in NK cells. Panels C and D show the corresponding results for monocytes, i.e. DE for 60 and 9 LATE genes detected in RNAseq and with both technologies, respectively.

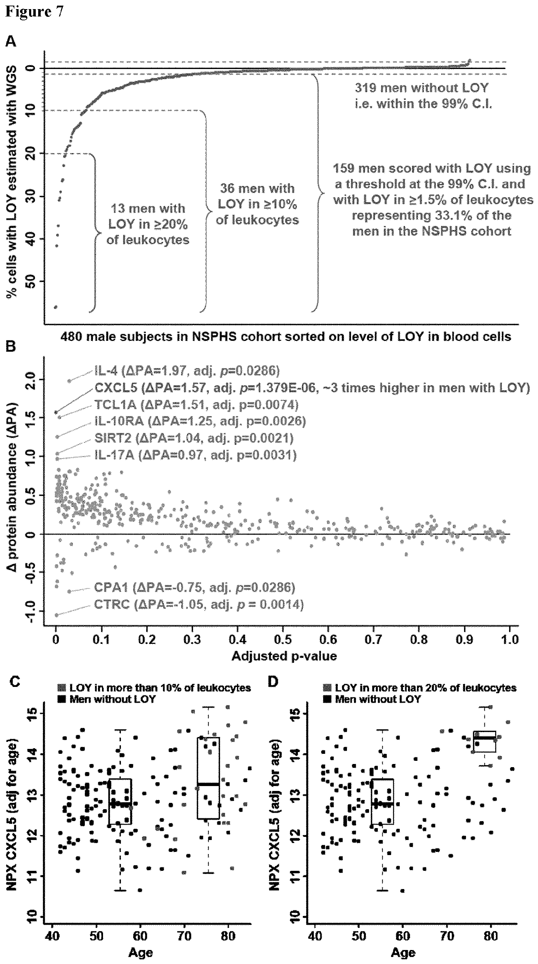

Figure 7 shows analyses of changes of plasma protein levels in men with LOY from the NSPHS cohort. Panel A shows result from analysis of LOY in blood DNA collected from 480 men studied with 30X whole genome sequencing. The level of LOY plotted on the Y-axis was estimated in each subject (represented by dots) by comparing the observed number of sequencing reads from the MSY in relation to the number of reads from the autosomes. The calculated ratio represents the percentage of cells with LOY, with a value close to zero in men carrying a Y chromosome in all studied cells. We used two thresholds for LOY scoring (i.e. ≥10% or ≥20% of cells with LOY) for identification of individuals with LOY for downstream analyses of protein abundance and comparisons with the 319 control subjects, defined using calculation of the 99% confidence interval (C.I.) of experimental variation. Panel B shows analysis of 424 plasma proteins in 13 men with LOY in ≥20% of leukocytes vs 319 controls. Protein levels were measured by the normalized protein expression (NPX). The difference in protein abundance (i.e. Δ PA) was estimated for each protein by subtraction of the observed mean NPX in the LOY group (adjusted for age) by the corresponding mean NPX in the controls. Abundance of each protein is visualized with grey dots and positive or negative values indicate higher or lower abundance in men with LOY vs controls, respectively. Panels C and D show individual measurements (NPX adjusted for age) of the CXCL5 for each of the subjects using the thresholds at ≥10% and ≥20% of leukocytes with LOY, respectively. The boxes on the X-axis are according to the mean age in each group.

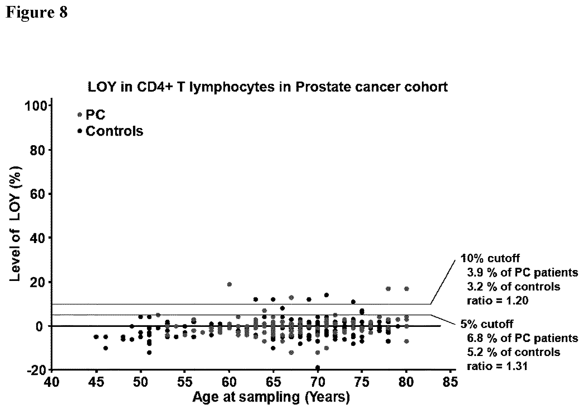

Figure 8 (which is related to Figure 2) shows a comparison of the level of LOY for each individual subject in sorted CD4+ T lymphocytes from the prostate cancer (PC) cohort, together with controls. On the Y-axis, two different cut-offs are shown for scoring of LOY at 5% and 10% of cells with this aneuploidy.

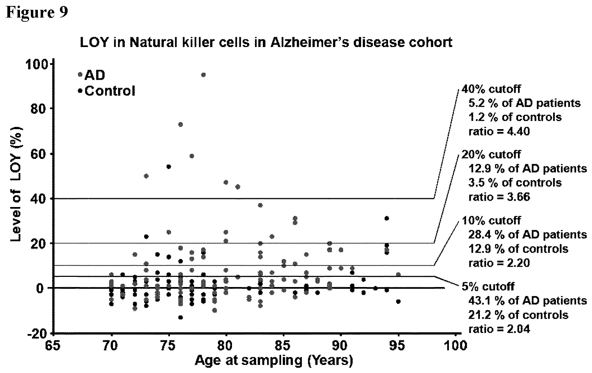

Figure 9 (which is related to Figure 2) shows a comparison of the level of LOY for each individual subject in sorted Natural Killer (NK) cells from the Alzheimer's disease (AD) cohort, together with controls. On the Y-axis, four different cut-offs are shown for scoring of LOY at 5%, 10%, 20% and 40% of cells with this aneuploidy.

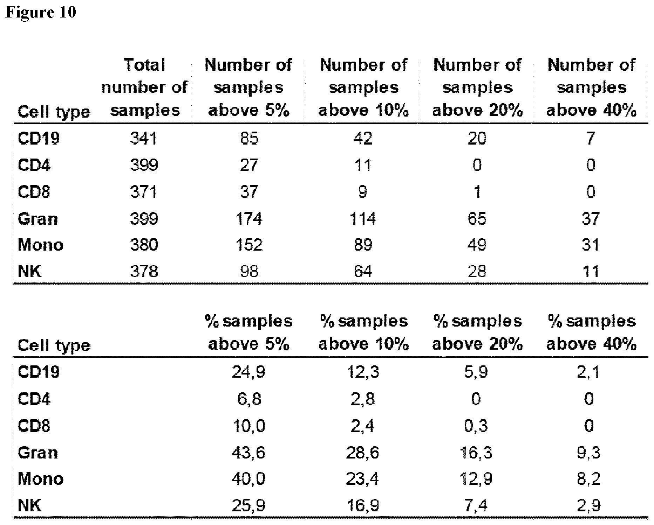

Figure 10 (which is related to Figure 2) shows the observed overall frequency of LOY in the six studied cell types collected from men diagnosed with AD, prostate cancer and controls. The table displays the number and frequency of samples showing LOY using four different cut-offs for LOY scoring.

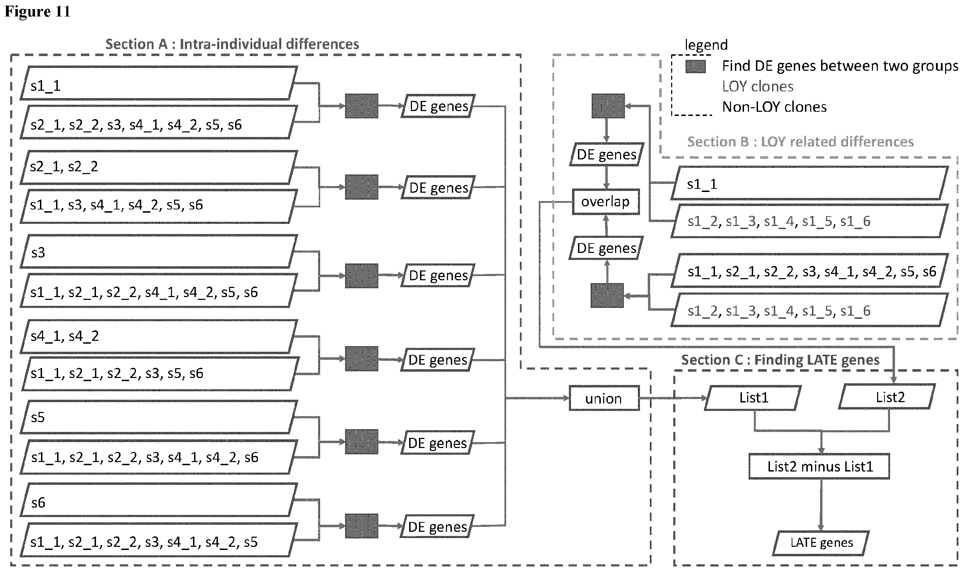

Figure 11 (which is related to Figure 5) shows a flowchart illustrating the analysis pipeline for finding genes showing **L**OY **A**ssociated **T**ranscriptional **E**ffect (LATE) in lymphoblastoid cell lines (LCLs). LCLs containing 100% LOY cells and no LOY cells are shown in red and black, respectively. Different cell lines derived from the same subject are shown with the same subject ID and an additional number; for instance "s2_1" and "s2_2" are two different cell lines derived from subject s2. The differentially expressed (DE) genes have been called using edgeR by comparing two groups of samples; this process is represented by blue boxes in the chart. In Section A, we compare each non-LOY subject (all cell lines, if there are multiple from the same subject) against all other non-LOY cell lines. The aim is to find the DE genes where the differential expression levels are due to the differences between individual subjects, and not the LOY status. The union of all DE gene lists from Section A generates List1, which is composed of 4175 genes. In Section B, we aim at finding DE genes related to LOY status by comparing: **i)** all LOY cell lines with all non-LOY clones; and **ii)** comparing five LOY clones of subject s1 with non-LOY clone from the same subject. The overlap of DE gene lists resulting from (**i**) and (**ii**) generates List2 (1531 genes). Finally, in Section C, the list of 904 LATE genes is made by taking the DE genes that exist in List2 but do not exist in List1. The rationale is to eliminate intra-individual differences in expression levels, from LOY-related variation.

### Detailed description of the invention

As presented above, disclosed herein are various methods for stratifying male subjects for immunotherapy or for predicting response to immunotherapy. Such methods may be used to provide, or to help guide, immunotherapy treatments, e.g. immunotherapy regimens.

The methods herein use LOY as a biomarker for predicted or likely response to an immunotherapy. Additionally, the present disclosure provides lists of genes which may further be used as biomarkers for predicted or likely response to an immunotherapy.

The term "immunotherapy" as used herein refers to any therapy which targets the immune system, or which uses an immunological agent or component. The immunotherapy may thus be therapy with an agent which targets, e.g. inhibits or activates, a component or element of the immune system, e.g. a cell or molecule (for example a protein) involved in an immune response of a subject. Immunotherapy is treatment with an immunotherapeutic agent. Generally, an immunotherapeutic agent may be an agent which stimulates a desired immune response in a subject (for example against a cancer). Such an agent may be immune checkpoint inhibitor. It may be an antibody. Whilst many antibodies are known as immune checkpoint inhibitors, antibodies may also be used in immunotherapy for other reasons, i.e. based on other mechanisms, or for different functional effects. Beyond antibodies, other molecules may modulate, or affect the immune system, or an immune response, e.g. a cytokine. An immunotherapeutic agent may further be a cell, notably an immune cell. Cells are used in adoptive cell transfer (ACT) therapy. For example, cytotoxic immune effector cells, such as T-cells (e.g. CTL, or CD8+ T-cells) or NK cells may be administered to a subject. Such immune cells may be modified (e.g. transfected, or otherwise genetically modified), e.g. to express heterologous antigen receptors, such as a TcR or CAR (chimeric antigen receptor).

The term "antibody" is used broadly herein to include any binding protein comprising, or based on, or derived from, an antigen binding site of an immunoglobulin molecule. The term thus includes natural or synthetic antibodies, whether monoclonal or polyclonal, as well as antibody fragments. The term thus includes any and all types of antibody molecule or antibody fragment. The term thus includes any molecule which is a full-length immunoglobulin molecule, or a fragment or derivative thereof. Accordingly, subsumed under this term is any antibody-type or antibody-derived molecule or fragment, or more generally any molecule which comprises an antigen-binding domain derived or obtained from an antibody (e.g. an immunoglobulin molecule, such as a native antibody), or based on the antigen-binding domain of an antibody. An antibody may alternatively be defined as immunological binding agent, or an immunointeractive agent. An antibody may accordingly be of any desired or convenient species, class or sub-type. It may be natural, derivatised or synthetic. Antibody derivatives, such as single chain antibodies, chimeric antibodies and other synthetically made or altered antibody-like molecules are well known in the art and widely described in the literature, and all are included. Antibody fragments include, for example, Fab, F(ab')₂, Fab' and Fv fragments, all of which are well-known and understood in the art.

An "immune checkpoint" is a molecule expressed on the surface of an immune cell, generally a CD4+ and/or CD8+ T-cell that fine-tunes an immune response. This may be by down-modulating or inhibiting an immune response. Checkpoint proteins keep the immune system in check by indicating to the immune system which cells are healthy and which cells should be destroyed. Checkpoint proteins act as a "brake" on the immune system by preventing T-cell activation. If a cell does not have sufficient checkpoint proteins on its surface it may be destroyed by the immune system. In the case of cancer cells, whilst there may be molecules signalling that the cell is cancerous, if there are enough checkpoint proteins on the cell surface, the cell may evade the immune response. Cancer cells are known to evade immune responses by way of immune checkpoints, and this underlies the well-known use of immune checkpoint inhibitors to treat cancer. Immune checkpoint proteins are well known in the art and include PD-1, PD-L1, PD-L2, CTLA-4, LAG-3, TIM-1,TIM-3, TIM-4, KIR, VISTA, B7-H7, B7-H3, B7-H4, B7-H6, 2B4 (CD244), ICOS, HVEM, CD160, gp49B, BTLA, SIRPalpha (CD47), CD48, B7.1, B7.2, ILT-2, ILT-4, TIGIT and A2aR. The term also includes fragments of these proteins.

Of particular interest herein is the immune checkpoint LAG3. As described in the Example below, studies have shown that LAG3 is substantially downregulated in leucocytes exhibiting LOY, particularly in NK cells.

An "immune checkpoint inhibitor" is an agent that inhibits the activity or function of an immune checkpoint protein, or its encoding nucleic acid. This may be an agent which binds to a checkpoint protein or to a receptor for a checkpoint protein. Inhibition of an immune checkpoint can block or otherwise neutralise or reduce inhibitory signalling, thereby to upregulate an immune response (e.g. an anti-cancer immune response). An immune checkpoint inhibitor may take various forms, e.g. it may be an antibody, a small molecule (e.g. small organic molecule), peptide, peptidomimetic, aptamer, a natural ligand or derivative thereof. It may be any agent that may bind to an immune checkpoint protein and/or inactivate or inhibit it, as well as an inhibitory RNA (e.g. RNA interference), anti-sense or other nucleic acid molecule that can inhibit expression and/or activity of a nucleic acid molecule, or nucleotide sequence encoding an immune checkpoint protein. An immune checkpoint inhibitor, such as antibody directed against (i.e. capable of binding specifically to) an immune checkpoint protein or an inactivated ("non-activating") or soluble or fusion protein form of the immune checkpoint protein may bind to the immune checkpoint protein on a cell and/or otherwise inhibit its action or functional effect. An immune checkpoint inhibitor may directly or indirectly inhibit the effect of an immune checkpoint. Many different immune checkpoint inhibitors have been described in the art, including a number of antibodies, in clinical trials or in clinical use. For example, various checkpoint inhibitors for use in the treatment of cancer are described in Creelan (2014) Cancer Control 21:80-89.

Examples of checkpoint inhibitors include: Relatlimab, an anti-LAG-3 monoclonal antibody (BMS-986016); TSR-033, a humanized IgG4 anti-LAG-3 monoclonal antibody; 25F7, a monoclonal antibody to LAG-3; Lirilumab, a monoclonal antibody to KIR; MBSA43, a monoclonal anti-TIGIT antibody; CL1-R2, a monoclonal antibody to CD160; and TTI-621 and TTI-622, antibodies for CD47. Any of these checkpoint inhibitors, or inhibitors like these, can be used. Thus, for example, antibodies against an immune checkpoint protein can be prepared according to techniques known in the art.

The immunotherapy may be for treating any condition responsive to an immunotherapy. That is, the condition may be any condition which benefits from the immunotherapy, e.g. from an upregulated immune response. Notably, the condition may be cancer. A large number of immunotherapies have been reported or proposed for treatment of various different cancers, including notably checkpoint inhibitors. Other conditions include conditions associated with a dysfunctional or dysregulated immune system, e.g. immunosuppression, or conditions associated with immunosuppression, or having immunosuppression as a component thereof, or autoimmune conditions as disorders, including age-related macular degeneration (AMD). Other conditions further include cardiovascular disease (CVD), cardiovascular events and conditions associated with CVD, platelet-type bleeding disorder 11, type 2 diabetes, multiple sclerosis, glaucoma, type 2 von Willebrand disease, Noonan syndrome, severe combined immunodeficiency autosomal recessive T-cell-negative/B-cell-positive/NK-cell-positive (SCIDBNK), T cell immunodeficiency, Beckwith-Wiedemann syndrome, Charcot-Marie-Tooth disease type 1C, extramammary Paget's disease, complement factor D deficiency, rheumatoid arthritis, psoriasis, inflammatory bowel disease, autosomal severe combined immunodeficiency disease, selective T cell defect, obesity, hypogonadism, and systemic lupus erythematosus, Mention may also be made of AD. In this regard various antibodies have been proposed and reported for use in the treatment or management of AD.

The term "cancer" is used broadly herein to refer to any malignant or pre-malignant neoplastic condition. Cancer cells may possess one or more characteristics typical of cancer-causing cells, such as uncontrolled proliferation, immortality, metastatic potential, rapid growth and proliferation rate, and certain morphological features. In some cases, cancer cells may exhibit such characteristics in part or in full due to the expression or activity of an immune checkpoint protein. A cancer may be in the form of a solid tumour, but cancer cells may exist alone in a subject. In certain embodiments haematological cancers are included. In other embodiments, the cancer is a non-haematological cancer. The cancer may be of any tissue organ of the body, and all known cancers and cancer types are included.

By way of representative example, the following cancers are listed, but it will be noted that for the methods involving male subjects and LOY, cancers of the female reproductive organs or genitalia are not included:
acute lymphoblastic leukaemia (ALL), acute myeloid leukaemia (AML), adrenocortical carcinoma, AIDS-related cancer (e.g. Kaposi sarcoma and lymphoma), anal cancer, appendix cancer, astrocytomas, atypical teratoid/rhabdoid tumour, basal cell carcinoma, bile duct cancer, extrahepatic bladder cancer, bone cancer (e.g. Ewing sarcoma, osteosarcoma and malignant fibrous histiocytoma), brain stem glioma, brain cancer, breast cancer, bronchial tumours, Burkitt lymphoma, carcinoid tumour, cardiac (heart) tumours, cancer of the central nervous system (including atypical teratoid/rhabdoid tumour, embryonal tumours, germ cell tumour, lymphoma), cervical cancer, chordoma, chronic lymphocytic leukaemia (CLL), chronic myelogenous leukaemia (CML), chronic myeloproliferative disorder, colon cancer, colorectal cancer, craniopharyngioma, cutaneous t-cell lymphoma, bile duct cancer, extrahepatic ductal carcinoma in situ (DCIS), embryonal tumours, endometrial cancer, ependymoma, oesophageal cancer, aesthesioneuroblastoma, Ewing sarcoma, extracranial germ cell tumour, extragonadal germ cell tumour, extrahepatic bile duct cancer, eye cancer (including intraocular melanoma and retinoblastoma), fibrous histiocytoma of bone, gallbladder cancer, gastric (stomach) cancer, gastrointestinal carcinoid tumour, gastrointestinal stromal tumours (GIST), germ cell tumour, gestational trophoblastic disease, glioma, hairy cell leukaemia, head and neck cancer, heart cancer, hepatocellular (liver) cancer, histiocytosis, langerhans cell, hodgkin lymphoma, hypopharyngeal cancer, intraocular melanoma, islet cell tumours, pancreatic neuroendocrine tumours, kaposi sarcoma, kidney cancer (including renal cell and wilms tumour), langerhans cell histiocytosis, laryngeal cancer, leukaemia (including acute lymphoblastic (ALL), acute myeloid (AML), chronic lymphocytic (CLL), chronic myelogenous (CML), lip and oral cavity cancer, liver cancer (primary), lobular breast carcinoma in situ (LCIS), lung cancer, lymphoma, macroglobulinaemia, Waldenström, melanoma, Merkel cell carcinoma, mesothelioma, metastatic squamous neck cancer with occult primary, midline tract carcinoma involving nut gene, mouth cancer, multiple endocrine neoplasia syndromes, childhood, multiple myeloma/plasma cell neoplasm, mycosis fungoides, myelodysplastic syndromes, myelodysplastic/myeloproliferative neoplasms, multiple myeloma, myeloproliferative disorders, nasal cavity and paranasal sinus cancer, nasopharyngeal cancer, neuroblastoma, non-Hodgkin lymphoma, non-small cell lung cancer, oral cancer, oral cavity cancer, oropharyngeal cancer, osteosarcoma, ovarian cancer, pancreatic cancer, pancreatic neuroendocrine tumours (islet cell tumours), papillomatosis, paraganglioma, paranasal sinus and nasal cavity cancer, parathyroid cancer, penile cancer, pharyngeal cancer, pheochromocytoma, pituitary tumour, plasma cell neoplasm/multiple myeloma, pleuropulmonary blastoma, pregnancy and breast cancer, primary central nervous system (CNS) lymphoma, prostate cancer, rectal cancer, renal cell (kidney) cancer, renal pelvis and ureter, transitional cell cancer, retinoblastoma, rhabdomyosarcoma, salivary gland cancer, sarcoma, Sézary syndrome, skin cancer, small cell lung cancer, small intestine cancer, soft tissue sarcoma, squamous cell carcinoma, squamous neck cancer with occult primary, metastatic, stomach (gastric) cancer, T-cell lymphoma, testicular cancer, throat cancer, thymoma and thymic carcinoma, thyroid cancer, transitional cell cancer of the renal pelvis and ureter, urethral cancer, uterine cancer, endometrial, uterine sarcoma, vaginal cancer, vulvar cancer, Waldenström macroglobulinaemia, and Wilms tumour.

Particular mention may be made of prostate cancer, skin cancer, lung cancer, CNS cancers, colorectal cancer, urogenital cancer, blood and lymphatic cancer, gastro-intestinal cancer, ear/nose/throat cancer, pancreatic cancer, and malignant melanoma. In the case of methods involving LOY determinations, particular mention may be made of prostate cancer, skin cancer, lung cancer, and CNS cancers.

As discussed above, methods for stratifying subjects may include assigning subjects to a treatment group. Additionally or alternatively, the methods may involve categorising subjects according to their LOY status, and in certain embodiments also biomarker expression levels. Response of the subjects to an immunotherapy may be assessed, and correlated to their LOY status and optionally biomarker expression levels. This may further be used in methods of predicting response or likelihood of response to an immunotherapy. Thus, such correlations may be made, or may be used, to establish a control against which LOY status and optionally biomarker expression levels may be compared to assign the subject to a treatment group, or to predict response or likelihood of a subject to respond to an immunotherapy. Such a control may for example be a threshold value. Thus, a pre-defined threshold may be determined for the method of predicting response, or for identifying a likelihood of response to an immunotherapy.

The methods herein may thus find utility for stratifying subjects for clinical trials, for therapy in the clinic, to help determine an appropriate treatment or treatment regimen for the subject.

Accordingly, the stratification or response prediction (etc.) methods herein may further comprise a step of determining an immunotherapy to be administered to the subject. Thus, based on results of the LOY, and in some cases biomarker determinations, a clinician may decide on the appropriate therapy to be administered to the subject, or to a sub-group of subjects, e.g. subjects which are LOY+ or which are LOY-. As indicated above, this may be that an immunotherapy is administered or is not administered. Alternatively, different immunotherapies may be administered to a subject, depending on the result of the determination, or prediction of response. In certain cases, it is determined that a subject is not likely to respond to an immunotherapy, a different type of therapy may be administered. Thus, for example in the case of cancer, a different type of therapy, or treatment with another type of anticancer agent may be determined for that subject, or group of subjects, such as chemotherapy, or radiotherapy, or surgery. The methods herein thus allow differential treatment to be determined for a subject. The methods may be used to guide or determine the treatment or clinical handling of a subject.

Hence, the methods may comprise recommending or prescribing an immunotherapy to the subject.

The methods herein based on a LOY determination comprise a step of determining a fraction of leucocytes that have lost chromosome Y in a sample from a male subject. A leucocyte is a white blood cell, or in other words a nucleated blood cell. The term thus includes various types of nucleated cells that may occur in blood or haematopoietic tissue. The leucocytes may thus be, or may comprise, agranulocytes (that is mononuclear leucocytes), or granulocytes (polymorphonuclear leucocytes). Agranulocytes include lymphocytes, monocytes and macrophages. Lymphocytes include B-cells, T-cells and Natural Killer (NK) cells. Granulocytes include neutrophils, basophils and eosinophils.

The various leucocytes types listed above may further be sub-divided into more specific leucocytes sub-sets, e.g. CD4+ T-cells (e.g. helper T-cells), CD8+ cytotoxic T-cells, or CD19+ B-cells.

In an embodiment, the leucocytes may be defined as immune cells, or immune effector cells.

The sample of leucocytes which is used in the methods may thus be any sample that comprises leucocytes, or a subset of leucocytes. The sample can be any sample that comprises leucocytes obtained from the male subject. The sample can be taken directly from the subject, and it may be processed for the LOY determination. Thus, the method may comprise a step of taking, or obtaining a sample from the subject. Non-limiting but suitable examples of sample include a blood sample, or a sample of haemopoietic tissue, such as bone marrow or spleen. For convenience the sample will typically be a blood sample. The blood sample may be processed to separate or isolate leucocytes, or a leucocyte subset therefrom, and therefore more generally the sample may be a blood or blood-derived sample which contains leucocytes, e.g. a PBMC sample.

Thus, in the methods herein the leucocytes may be separated from a blood sample, or other biological sample, obtained from the male subject. The sample taken from the subject may be processed, e.g. by a separation step to provide the leucocyte-containing sample. In other words, the sample that is used in the determination step may be any sample that contains leucocytes obtained from the subject. Alternatively expressed, this means that the determination may be performed on cells from a biological sample obtained from the subject, and this encompasses that for the determination step the cells may be taken or separated from the sample which is taken from the subject, e.g. prior to the determination being carried out, or that the determination may be performed in a selective manner on a particular set of cells (i.e. a sub-set of cells) in the sample.

A biological sample obtained from a subject may thus be subjected to a separation step to separate a subset of cells from the sample for the determination step. The methods herein may include a separation step of the biological sample prior to carrying out the determination. The determination of the fraction of cells that have lost Y may thus comprise performing the determination on a subset of cells from the biological sample (i.e. determining the fraction of cells from a subset of cells from the biological sample that have lost chromosome Y).

Such a separation step may be used to separate or isolate a particular leucocyte cell type or a particular subset of leucocyte cells from a biological sample.

Methods for separating or sorting cells are well known in the art, e.g. for separating a particular leucocyte type from a mixture of cells in a sample such as blood which contains a mixture of different types of cell. This will depend on the nature or type of cell to be separated, but may for example be done by centrifugation or other separation techniques based on the physico-chemical properties of the cell, or by positive and/or negative selection based on cell markers for a particular cell type.

Leucocytes may be separated from a blood or other biological sample for the determination step. The methods may be performed on a sample containing multiple types of leucocytes, or it may be performed on a sample containing just one specific type of leucocyte, e.g. NK cells, or T-cells, or a more specific sub-type, e.g. CD4+ T-cells. Alternatively, the sample may contain more than one type of leucocyte e.g. it may contain all granulocytes, or all mononuclear cells, etc. Further, the method may be performed using samples of different leucocytes for a subject, e.g. particular types or sub-types may be investigated separately (for example in parallel).

As noted above, methods for separating different types of leukocytes are well known in the art. For example PBMC and granulocytes may be separated by Ficoll-Paque centrifugation (Amersham Biosciences, Uppsala, Sweden) and T-cells e.g. CD4+ T-cells may be separated (e.g from the PBMC fraction) by positive and/or negative selection based on cell-surface marker expression (e.g. CD4 expression). Kits and materials for performing such separations are commercially available (e.g. the CD4+ T cell Isolation Kit and CD4 microbeads from Miltenyi Biotech, Auburn, CA, USA). However, the separation is not limited to such methods and may be performed by other means e.g by FACS sorting etc.

The determination of the fraction of leucocytes that have lost chromosome Y can be performed according to various analysis methods and techniques. Generally, DNA and/or RNA from the cells in the sample is analysed in order to determine presence or absence of chromosome Y in the cells. Thus, DNA and/or RNA may be obtained from the cells to be analysed. For instance, polymerase chain reaction (PCR) primers can be used to detect the presence or absence of any DNA sequence in chromosome Y and where the absence of the chromosome-Y-specific DNA sequence is regarded as LOY. Correspondingly, techniques can be used to detect the presence or absence of mRNA transcribed from active genes present on chromosome Y. Absence of any significant detectable amounts of mRNA could then be regarded as LOY. Alternatively, the detection of LOY can be done on protein basis instead of or as an alternative to DNA and/or RNA analysis. In such a case, the presence or absence of a protein encoded by a gene on chromosome Y can be used to discriminate between those cells that have an intact chromosome Y and those cells that have lost chromosome Y.

In an embodiment, LOY may be determined in an individual subject by single cell analysis, to detect whether or not mRNA transcripts are present, or the amount thereof, from the male specific region of chromosome (MSY). Analogously a single cell analysis may be performed to measure DNA or protein.

Further non-limiting examples of analysis methods that can be used include single-nucleotide polymorphism (SNP) array analysis, RNA-assays, gene expression analysis, protein assays, epigenetic assays and various genetic methods, such as sequencing, comparative genomic hybridization (CGH) arrays, methylome assays and cytogenetic methods. It is also possible to visually detect LOY using microscopy methods.

Sequencing methods that can be used include basic and advanced sequencing methods, such as Maxam-Gillbert sequencing, Chain-termination methods, Shotgun sequencing and bridge PCT. Also, or alternatively, next-generation sequencing methods could be used to detect LOY and determine the fraction of cells that have lost chromosome Y. Examples of such next-generation sequencing methods include massively parallel signature sequencing (MPSS), polony sequencing, 454 pyrosequencing, Illumina sequencing, SOLID sequencing, Ion Torrent semiconductor sequencing, DNA nanoball sequencing, Heliscope single molecule sequencing, single molecule real time (SMRT) sequencing, nanopore DNA sequencing, tunneling currents DNS sequencing, sequencing by hybridization, sequencing with mass spectrometry, microfluidic Sanger sequencing, microscopy-based techniques, RNAP sequencing and *in vitro* virus high-throughput sequencing.

In a particular embodiment, the determination of the fraction in step S1 is based on sequencing of a DNA region present on chromosome Y.

In another particular embodiment, the determination of the fraction of leucocyte with LOY may be based on SNP array analysis using data points derived from a pseudoautosomal region 1 (PAR1) region of chromosomes X and Y. In particular, the fraction may be determined based on a quantification of the data points derived from the PAR1 region by using a correlation between a Log R Ratio (LRR) in the PAR1 region of chromosome Y and an absolute deviation from an expected diploid B-allele frequency (BAF) value of 0.5. Such a method is described and used in WO 2015/052190 and WO 2015/121317.

As used in the Example below, LOY may be determined by SNP array analysis using SNP array probes located in the male-specific region of chromosome Y (MSY). A continuous mLRRY analysis may be performed as described in Forsberg et al., 2014, Nature Genetics 46, 624-628. Statistical analyses may be performed using the continuous estimate of LOY (mLRRY).

In the methods herein a subject may be determined to be LOY+ or LOY-. In other words, it is determined whether the subject has or exhibits loss of chromosome Y in leucocytes, or does not have or exhibit loss of chromosome Y in leucocytes. Such a determination, or assignment into LOY+ or LOY- sub-groups, involves comparing the determined fraction to a threshold, or more particularly a predefined threshold.

Further, certain methods herein may comprise predicting response or likelihood of response to immunotherapy based on comparing the fraction of leucocytes in the sample that have lost chromosome Y to a threshold or more particularly a predefined threshold.

A predefined threshold may thereby be employed to differentiate male subjects that are LOY+ from those that are LOY-, or those male subjects who are predicted or likely to respond to an immunotherapy to those that are not.

The predefined threshold, to which the determined fraction of leucocytes is compared may be determined by the person skilled in the art based on various parameters according to methods and principles known in the art, including for example determining the fraction of cells that have lost chromosome Y in control or reference subjects, including in multiple male subjects, e.g. cohorts or groups of reference or control subjects. The control or reference subject or cohort may be selected based on the method that is being performed. The threshold may depend on the technology that is used for the LOY determination. Thus, where LOY is scored from DNA in NGS data or SNP-arrays, typically a threshold may be determined from a cohort or population of male subjects. Thus, effectively a control or reference population or cohort of subjects may be used to establish a control or threshold value for determining the LOY status of the male subject. Thus, there may be an "external" control, i.e. the control may be based on a subject or group of subjects which is external to the subject or group of subjects under test in the method. In other cases, for example where the LOY determination is based on a single cell analysis a population or group-based threshold may not be required. A threshold may be set simply on the basis of the fraction, or level, or value for the cells detected to have LOY. Thus, in an embodiment, the subject himself may provide the control. In other words there can be an internal control.

Thus, in one embodiment for assessing LOY+ versus LOY-, the threshold may be selected based on population studies of male subjects, or it may be determined by determining the fraction of leucocytes that have lost LOY in a cohort of healthy male subjects, and/or male subjects below and/or above a certain age. In another embodiment, e.g. where a single cell analysis is used, as noted above, there may be no need for a population-based or external control-based threshold. A male subject may be scored as LOY+ or LOY- based on a determination performed on a sample from the subject himself, e.g. depending on whether or not MSY DNA, mRNA or protein may be detected in a subject himself. Accordingly, a threshold may simply be set for a fraction, e.g. percentage, of cells that have LOY. A threshold may accordingly be set as zero - it can simply be determined whether or not any cells can be detected that do, or do not have LOY. If any MSY material can be detected, the subject may be determined (scored) to be LOY+, and if no MSY material can be detected, the subject may be determined (scored) to be LOY-. In other words the threshold may simply be presence or absence of MSY material, or presence or absence of cells with LOY. Alternatively, a minimum level of detectable cells which are LOY-may be set, equal to or above which a subject may be determined to be LOY-.

For methods that involve predicting or identifying likelihood of response, the predefined threshold may be set by using control subjects that have responded and/or not responded to the immunotherapy in question. The predefined threshold can be set to discriminate between these two groups.

In practical terms, as noted above, the threshold which is used may depend on the technique which is used to determine the loss of the Y chromosome. As described further below in the Example, the degree of LOY may be calculated from the median log R ratio values (measuring copy number) for genotyping using an array of probes in the male-specific region of chromosome Y (mLRR-Y). The mLRR-Y value may be used as a proxy for LOY, which is itself a proxy for the degree of mosaicism i.e. the percentage of cells that have lost the Y chromosome. Thresholds may be set depending on mLRR-Y values. Specific mLRR-Y values are of course applicable only to the SNP-array technique and measurements and calculations as used. However, the thresholds may be set at the level of mosaicism (i.e. at the level of LOY) where the percentage of cells affected by LOY is not in the range of experimental variation and this is applicable to any means by which the LOY is measured or assessed (i.e. any technique or method for determining LOY). For example the threshold may be set at the level where the most robust statistical results are obtained (e.g. the most robust regression results, as described below). However, the threshold may be varied and can be set at a less stringent, or lower, level, for example the lowest value in the noise distribution (variation) may be used as the threshold. Thus, experimental variation, or experimental background noise, or more particularly the distribution thereof, may be used to set the predefined threshold for determination of LOY. The limit of experimental variation, and hence the threshold, may be set at different levels of stringency.

The lower 99% confidence interval (CI) of the distribution of expected experimental background noise (i.e. experimental variation) may be used as a pre-defined threshold to detect or determine LOY and this may be taken as representing an embodiment of a predefined threshold according to the methods herein. Accordingly, in an embodiment the method may be performed using the 99% confidence limits of a data set and the predefined threshold may be set according to (i.e. at or as) the lower limit of 99% confidence (i.e. using the lower 99% Cl limit of the distribution of experimental variation (or alternatively expressed, of the distribution of experimental background noise) as the predefined threshold).

Accordingly, in one embodiment a predefined threshold may be the lower 99% Cl of a LOY determination (i.e. a LOY frequency determination). For example, this may be a LOY determination (or more particularly a LOY frequency determination) in a population of male subjects generally being assessed for LOY, or being assessed for response prediction or likelihood, or being assessed for disease development.

The data which is analysed to determine the fraction of cells with LOY and which may be used to set the predefined threshold may be corrected to allow or correct for any factors which may affect or confound the data, for example for variation between cohorts of data, and this may be performed according to principles well known in the art.

Thus, thresholds used in practice in a particular method of LOY determination may be translated into, or used to determine, a generally-applicable threshold which is not specific for or dependent upon a particular technique or method. For example, the percentage of cells which have lost chromosome Y may be calculated from the mLRR-Y values and a threshold may be set according to the percentage of cells in the sample, or more particularly the percentage of cells analysed, which are determined to have LOY (i.e. be affected by LOY).

A non-limiting but illustrative value of the predefined threshold is about, or at least, 5, 7, 8, 10, 13, 18, 20, 25, 30, or 40 %, in terms of the % of cells in the sample. The fraction of cells which have lost chromosome Y may thus be determined as a percentage of cells.

The predefined threshold may be selected to be in a range of from about 5 % to about 50 % (i.e. 5 to 50 % of cells). This means that if at least 5 % to 50 % of the cells from the sample have lost chromosome Y then the male subject may be classified as LOY-, or may be predicted to respond or be likely to respond to an immunotherapy, or may be is predicted to have an increased risk of developing AD or prostate cancer.

Other ranges for threshold values include 5-40, 5-35, 5-30, 5-25, 5-20, 5-15, or 10-40, 10-35, 10-30, 10-25, 10-20, or 10-15% of cells from the sample that have lost chromosome Y, or more particularly of cells analysed. In other embodiments the range may be from any one of 1, 2, 3, 4, 5, 6, 7, 8, 9 or 10 to any one of 12, 15, 20, 25, 30, 35, 40, 45, or 50% cells from the sample that have lost chromosome Y, or more particularly of cells analysed. Other ranges include 1-12, 1-10, 5-15, 5-12, 5-10, 7-15, 7-12, 7-10, 8-15, 8-12, 8-11, 9-12, and 9-11, or 1-8, 1-7 or 1-5 %. In one representative embodiment the threshold may be about 10% of cells. In another representative embodiment the threshold may be about 13% or about 13.1% of cells, as determined from the study below. The ranges, or thresholds may depend on the method which is being performed and the assessment which is made.

As noted above, a determination of LOY status may be made simply based on whether or not the presence of chromosome Y can be detected in cells. In some cases the threshold may simply be the presence or absence of cells which are LOY- (i.e. that lost chromosome Y). Thus, in another embodiment, a threshold may be 0% cells that have lost chromosome Y, or can be detected to have lost chromosome Y. 0% cells with LOY may indicate the subject to be LOY+. Conversely, the threshold may be 0% cells that have not lost chromosome Y (i.e. that contain chromosome Y, or which can be detected to contain chromosome Y), and this may indicate the subject to be LOY-.

Estimations of fractions (e.g. percentages) of leucocytes that have LOY may be determined from the obtained data using software packages that are known and available in the art. For example, software for estimating percentage of cells that have LOY based on SNP-array data is publically available (e.g. as used in the Example below) and described for example in in WO 2015/052190 and WO 2015/121317.

As noted above, the methods based on a LOY determination may further include determining the expression, or more particularly the level or amount of expression, of one or more biomarkers in a sample from the subject. The sample may be the same sample, or a different sample from the subject. The biomarkers may particularly be LATE genes as defined herein, that is a gene associated with LOY, or more particularly a gene which exhibits a LOY Associated Transcriptional Effect.

Since the level of expression may be determined at the protein or nucleic acid level, and further since altered gene expression may be detected at the level of the proteome in the plasma, the sample which is used for this expression analysis need not be a sample of, or containing leucocytes, and may be any sample of body fluid, tissue or cells from the subject. It thus may be any clinical sample that may be obtained from the subject. It may thus be a serum or plasma sample. However, in an embodiment the sample may be sample of or containing leucocytes, or subset of leucocytes, as defined and described above.

The biomarker may be any one or more of the 134 genes listed in Table 4. More particularly it may be any one or more of the 95 genes listed in Table 2 below.

**Table 2 - list of 95 LATE genes**

| | | | |
|---|---|---|---|
| *ABI3* | *FXYD5* | *MERTK* | *RPS27* |
| *ALCAM* | *GALNT2* | *MIIP* | *RPS29* |
| *ANXA1* | *GPR150* | *MRAS* | *RPS5* |
| *APBA3* | *GPX1* | *MT2A* | *RPSA* |
| *ATF3* | *HERC6* | *MX1* | *S100A12* |
| *CADM1* | *HLA-DPA1* | *MYOM2* | *SLC22A18* |
| *CCDC85B* | *HLA-DPB1* | *NCOA4* | *SLC25A6* |
| *CCL3* | *HLA-DQA1* | *OAS2* | *SLC9A3R1* |
| *CCL5* | *HLA-DQB1* | *OASL* | *snoRNAs (38 genes)* |
| *CD3D* | *HLA-DRA* | *P2RY10* | *SORL1* |
| *CD3G* | *HLA-DRB5* | *PADI2* | *ST14* |
| *CD7* | *IFIT1* | *PARP12* | *TCL1A* |
| *CD74* | *IFITM3* | *PCDHGB5* | *TGIF2* |
| *CD99* | *IL15* | *PFKFB4* | *TIGIT* |
| *CDKN1C* | *ISG15* | *PPBP* | *TTC9* |
| *CFD* | *JAK3* | *PRLR* | *USP18* |
| *CLPTM1L* | *KLRG1* | *PRSS57* | *USP9Y* |
| *CSF1R* | *LAG3* | *PYCARD* | *VASP* |
| *CSF2RA* | *LAGE3* | *PYHIN1* | *ZAP70* |
| *CTNNB1* | *LEP* | *RANBP2* | *ZKSCAN1* |
| *DDX58* | *LILRA1* | *RPL10A* | |
| *EGR1* | *LILRB2* | *RPL34* | |
| *EPSTI1* | *LITAF* | *RPL7* | |
| *FCER1G* | *LY6E* | *RPS12* | |
| *FLT3LG* | *MARCO* | *RPS15* | |

In an embodiment the biomarker may be any one or more of LAG3, LY6E, ANXA1, CSF1R, APBA3, CSF2RA, EGR1, JAK3, ISG15, IL15, CD3D, CD3G, CADM1, PYCARD, USP18, USP9Y, LILRB2, LILRA1, VASP, ZAP70, KLRG1, P2RY10, CFD, CCL3, FCERG, HLA-DRA and CCL5.

In an embodiment, the biomarker may be any one or more of the PAR gene CD99, the MSY genes *EIF1AY* and *RPS4Y1,* or the autosomal gene *LY6E.*

In an embodiment, the biomarker may be any one or more of: *ABI3, AKAP17A, CCDC85B, CCL3, CCL5, CD2, CD3D, CD3G, CD99, EIF1AY, FBXO6, FCER1G, HLA-DRA, KLRG1, LY6E, PABPC1, RPL7,RPS4Y1, CD99, CSF2RA, EEF1B2, EIF1AY, LITAF, LY6E, RPS4Y1, S100A12* and *TYMP.*

In particular, where the methods are performed on NK cells, i.e. where the sample is a sample of or containing NK cells, the biomarker may be any one or more of: *ABI3, AKAP17A, CCDC85B, CCL3, CCL5, CD2, CD3D, CD3G, CD99, EIF1AY, FBXO6, FCER1G, HLA-DRA, KLRG1, LY6E, PABPC1, RPL7* and *RPS4Y1.* Similarly, where the methods are performed on monocytes, i.e. where the sample is a sample of or containing monocytes, the biomarker may be any one or more of: *CD99, CSF2RA, EEF1B2, EIF1AY, LITAF, LY6E, RPS4Y1, S100A12* and *TYMP.* Where the methods are performed on CD19+ B lymphocytes, i.e. where the sample is a sample of or containing CD19+ B lymphocytes, the biomarker may be TCL1A.

Thus, one or more of the biomarkers may be assessed, e.g. 2, 3, 4, 5, 6, 7, 8, 9, 10, 15, 20, 25, 27, 30, 40, 50, 60, 70, 80 or 90 or more, in any combination.

In certain embodiments, LAG3 and/or LY6E are assessed. As described in the Example below we have particularly shown that LAG3 is substantially downregulated in leucocytes exhibiting LOY. In particular, LAG3 may be downregulated in NK cells. Accordingly, in an embodiment the level of expression of LAG3 is determined. In another embodiment LAG3 may be assessed in NK cells. In further embodiments expression of LAG3 and/or LY6E may be determined in combination with any one or more of the above-mentioned biomarkers, or lists of biomarkers.

The level of expression of the biomarker, whether of a biomarker gene or a biomarker protein, may be determined by methods well known and widely used in the art.

Expression is the process by which information from a gene is used in the synthesis of a functional gene product. These products are often proteins, but in non-protein coding genes such as transfer RNA (tRNA) or small nuclear RNA (snRNA) genes, the product is a functional RNA. There are several steps in the gene expression process including transcription, RNA splicing, translation, and post-translational modification of a protein. Thus, determining the level of expression may refer to determining the level of any one of these processes. Furthermore the level of expression may be affected by at the gene level for instance copy number variants (CNVs). A difference in the copy number of a gene can increase or decrease the level of that gene's activity. For example, when a copy of a gene is duplicates, the cell may produce twice as much protein as compared to a normal cell.

The level of protein expression may be determined by determining the total amount of a particular protein present in a cell, including protein on the cell surface and intracellular protein. Alternatively, the level of protein expression may be measured by determining the protein expressed on the cells, i.e. on the cell surface. The cell may be any cell in the sample, and may therefore depend on the sample, but in an embodiment the cell may be a leucocyte (i.e. as discussed and defined above). Thus differential protein expression on a cell surface may be determined. Various methods for measuring the total level of a protein in a cell and cell surface protein levels are known to those skilled in the art. In a particular embodiment, the level or amount of the protein at or on the cell surface is determined. In another embodiment, that level of protein may be determined outside the context of a cell, for example in a body fluid or sample such as serum or plasma, or in a cell or tissue lysate or extract, etc. This amount or level may be compared to a control level, as discussed further below. Methods and reagents for determining protein expression are discussed further below.

In another embodiment, the expression of the biomarker(s) may be determined by determining or analysing nucleic acid in the cells. Methods for detecting, analysing and quantifying nucleic acids, including specific nucleic acids (i.e. sequence-specific detection or analysis etc.) are known in the art such as methods using nucleic acid hybridisation techniques including: southern blotting; northern blotting and fluorescence in-situ hybridization or polymerase chain reaction (PCR) including variants of PCT such as; reverse transcription polymerase chain reaction, real-time or quantitative PCR (qPCR), hot-start PCR, nested PCR or multiplex PCR. Accordingly, to determine a nucleic acid encoding a protein in question probes or primers may be used, more particular a hybridisation probe or a primer (e.g. a primer for an amplification process such as, but not limited to, PCR) may be used which comprises a nucleotide sequence capable of binding (hybridising) specifically to nucleic acid molecule (e.g a mRNA or a DNA molecule) encoding the protein. The probe or primer may be provided with a detectable label or reporter group, or the probe and/or primer may be capable of taking part in signal-giving reaction to generate a signal which may be detected in order to detect the encoding nucleic acid, in order to report on the level of expression of the protein

In general terms a control (e.g. a control level) is the material (e.g. cell or sample) or value (e.g. level) against which the test material (e.g. test cell or test sample) or test level etc. is compared (e.g. to determine if there has been a change, e.g. decrease or increase). In the methods herein, the determined level of expression of the biomarker(s) is compared to the expression level of a control. The control may thus be a reference level or reference value determined for the control level. In the context of stratifying subjects for immunotherapy based on likely or predicted response, or for predicting response or likely response, the control level may be a pre-determined level, determined by comparing reference or subjects who have responded to the therapy to those who have not, analogously to discussion above in relation to methods based on LOY determinations. This may be used to set or determine a pre-defined threshold value. The threshold may be used to delimit (or discriminate) predicted responders from predicted non-responders. Depending on the biomarker in question, different thresholds may be set, and depending on whether the biomarker is up-or down-regulated, a level equal to or above, or equal to or below, a given threshold may be used to stratify the subjects, and/or to assign them to a predicted responder or predicted non-responder group, and/or to a treatment group for therapy, e.g. for the immunotherapy.

In another embodiment, the subjects may be stratified based on a comparison to control which is a "normal" level or value, e.g. a predetermined level or value for the normal level of expression of a biomarker in a healthy subject Such a predetermined level or value may be determined in a representative sample of normal cells, or a sample from a healthy subject. Thus the control level of expression may be the level of expression in a normal cell, or a level of expression determined for a population of normal cells, or a cell or cells or other sample from a healthy subject. Analogously, a control level (or load) of genetic aberrations may be the level determined or detected in a normal cell. Representative normal samples (e.g. for a particular cell type and/or subject type) may be studied and used to determine a representative or normal value.

In the context of determining whether there is an increase or decrease in the level of expression of the biomarkers, the control level (e.g. for a particular biomarker in a particular cell type) may be determined for a cohort or population of subjects and may represent the range of variability found within the cohort or population. Thus the control value may represent the range of variability for that biomarker in that cell type in that subject type. The control level may accordingly be provided as a range, or a value, of limits of variability. In an embodiment, the control level may be a level determined from a normal or healthy cohort of subjects. A control level determined from a normal cohort may be used as a predefined threshold level for comparison against a level determined from a subject.

As indicated above, where more than one biomarker (i.e. biomarker panel or combination) is assessed, a biomarker signature or profile is obtained for the subject which may be compared to a control biomarker signature or profile, i.e. a signature or profile determined or obtained from a control or reference subject or cohort or population of subjects, analogously to the discussion above.

Subjects may be assigned for immunotherapy based on the biomarker determination and comparison to control steps, in combination with the LOY determination. The discussion above in relation to stratification and determination of therapy applies analogously to the methods which also comprise determining LATE gene biomarker expression levels. Accordingly, the LOY-based, and optionally biomarker-based, stratification or response prediction (etc.) methods herein may further comprise a step of determining an immunotherapy to be administered to the subject. Thus, a clinician may decide on the appropriate therapy to be administered to the subject. As indicated above, this may be that an immunotherapy is administered or is not administered. Alternatively, different immunotherapies may be administered to a subject, depending on the result of the determination, or prediction of response. In certain cases, it is determined that a subject is not likely to respond to an immunotherapy, a different type of therapy may be administered, etc., as discussed above. The methods herein thus allow differential treatment to be determined for a subject. The methods may be used to guide or determine the treatment or clinical handling of a subject.

Hence, as above, the methods may comprise recommending or prescribing an immunotherapy to the subject.

A detection agent may be used to detect the level of expression of a biomarker. The detection agent may accordingly be directed towards a nucleic acid or a protein as the analyte which reports on (or which allows determination of) the level of expression of the biomarker. Thus, a detection agent is an agent which binds to a protein to be detected (whose expression level is to be determined) or to its encoding nucleic acid and is capable of disclosing the presence of the protein or nucleic acid. In other words, by detecting the binding of the detection agent to the protein or nucleic acid, the protein or nucleic acid may be detected. The amount or level of the protein or nucleic acid may be determined. A set of detection agents may be used, each capable of binding to a different biomarker (or its encoding nucleic acid).

The detection agent may comprise an analyte-binding partner (more particularly an analyte-specific binding partner) which binds to or is capable of binding to (or alternatively expressed, with binding activity or affinity for, e.g. with specificity for) the target analyte and a detectable moiety (e.g. group or domain which may be coupled or linked directly or indirectly to the binding partner) and which directly or indirectly allows detection of the analyte, e.g. a detectable label or reporter group. Thus the detectable group may be directly or indirectly detectable. A directly detectable label may, for example, be a spectrophotometrically or optically detectable label e.g. fluorescent tag or a radionuclide. An example of an indirectly detectable agent may be a nucleic acid or oligonucleotide label or tag which is detected in an amplification or other nucleic acid-based reaction (e.g. an enzymatically catalysed reaction such a ligation or primer-based polymerase extension reaction), or an enzyme or enzyme substrate which requires incubation with a substrate or an enzyme to generate a signal. Accordingly, a detectable label or reporter may be directly or indirectly signal-giving.

The analyte-binding partner can be for example a nucleic acid probe or primer or it can be a specific binding partner for a biomarker protein. A binding partner may be any moiety, e.g. any compound, molecule or entity having the ability to bind to analyte, protein or nucleic acid, in question. In particular the binding agent may bind specifically to the analyte. By binding specifically is meant that the binding partner is capable of binding to the protein or nucleic acid in a manner which distinguishes it from binding to a non-target molecule. Thus, binding to a non-target molecule may be negligible or substantially reduced as compared to binding to the target protein or encoding nucleic acid. A binding partner may thus be any moiety having a binding affinity for a biomarker protein.

A binding partner may be proteinaceous or non-proteinaceous. Where the target analyte is the protein (i.e. the biomarker protein to be detected), binding partners may thus be selected from proteins or polypeptides such as antibodies or fragments or derivatives thereof, a combinatorially derived polypeptide from phage display or ribosome display or any other peptide display system, a ligand for the protein or a fragment or derivative thereof (e.g. a naturally occurring or endogenous ligand or a fragment or derivative thereof) including for example non-agonistic fragments of the native ligands (e.g. polypeptides comprising a portion of the native or endogenous ligands), or a nucleic acid molecule, such as an aptamer, or combinations thereof.

In one embodiment, the binding agent is a protein, preferably an antibody, including derivatives or fragments thereof as discussed above.

As noted above the label or reporter can be any detectable label or reporter group. Such labels and reporter groups are known and widely reported in the art, as are assays and methods for detection based on the use of such detection agents. Thus the detection may comprise or may be based on an immunoassay, including immunohistochemistry.

In particular embodiments the label may be spectrophotometrically or optically (including visually) detectable e.g. a colorimetric or fluorescent label. In other embodiments the label may be a radiolabel.

Any convenient or desirable assay format may be used, including particularly any of the known formats described for immunoassays for protein detection, or probe/primer based nucleic acid assays, e.g. hybridisation assays based on specific probes, including padlock probes, allele-specific probe etc. Various techniques for detecting nucleic acids *in situ* in a sample are known, as are immunohistochemical or other techniques for detecting proteins expressed on cells.

The level of binding of detection agent correlates with the amount of analyte in the sample thus determining the level of binding of the binging partner allows the determination of the amount or level of analyte in the sample.

The genetic analysis can however be performed according to various analysis methods and techniques. Generally, DNA and/or RNA from the cells in the biological sample is analysed in order to determine the presence and/or amount of a nucleotide sequence corresponding to the biomarker gene. Thus DNA and/or RNA may be obtained from the cells to be analysed, e.g. from cells in the sample, or from a fraction or subset of cells in the sample. Various nucleic acid analysis techniques, e.g. PCR-based or sequencing methods are described above, and any of these may be used.

The invention will now be described in more detail in the Example below, with reference to the Figures and Tables herein.

### Example 1

### WET LAB METHODS

### 1. Sample collection

Blood samples for DNA and mRNA analyses were collected from 408 male subjects in Uppsala, Sweden and in Kraków, Poland. In Uppsala, samples from the cohorts Uppsala Longitudinal Study of Adult Men (ULSAM), Alzheimer's disease cohort (UAD) and controls (M) were collected during January 2015 to May 2018, at the Geriatric/Memory Clinic, Uppsala Academic Hospital. In Kraków, samples from prostate cancer patients (KP) were collected from March 2015 to May 2018 at the Centre of Oncology, Maria Sklodowska-Curie Memorial Institute, Kraków Branch and the Department and Clinic of Urology of the Jagiellonian University Collegium Medicum in Kraków. Samples from patients with Alzheimer's disease (AD) were collected from January 2017 to May 2018 at the Clinic of Internal Diseases and Gerontology of the Jagiellonian University, Collegium Medicum in Kraków. Control samples (M) were collected from December 2015 to May 2018 from the general population of Kraków and Uppsala. Blood samples for DNA and protein analyses were collected from 480 male participants of The Northern Swedish Population Health Study (NSPHS) during 2006 and 2009 (Assarsson and Lundberg, 2017). The criteria for recruitment of prostate cancer patients was advanced stage prostate cancer; i.e. Gleason grade 7 or higher. The prostate cancer patients were recruited before treatment, or during the first stage of treatment. As for Alzheimer's disease, patients with ongoing clinically and radiologically confirmed diagnosis were recruited. The clinical stage for recruited Alzheimer's patients was intermediate or severely advanced disease. Written informed consent was obtained from all individuals included in the study. The research was approved by the local research ethics committees of Uppsala Region in Sweden (Regionala Etikprövningsnämnden, Dnr 2013/350, Dnr 2015/092, Dnr 2015/458, Dnr 2015/458/2 with update from 2018) and by the Bioethical Committee of the Regional Medical Chamber in Kraków, Poland (NR: 6/KBL/OIL/2014).

### 2. Preparation and sorting of blood cells with FACS

We implemented two strategies for preparation of blood cells for sorting using Fluorescence Activated Cell Sorting (FACS), as described in sections 2.1 and 2.2 below.

### 2.1 Isolation and labeling of peripheral blood mononuclear cells (PBMCs) for sorting of T- and B lymphocytes as well as NK cells.

16 ml of whole blood were collected into two BD Vacutainer^{®} CPT^{™} Mononuclear Cell Preparation Tubes (BD). Blood samples were centrifuged within 2 hours of blood collection, following manufacturer's instructions. Isolated peripheral blood mononuclear cells (PBMCs) were stained with 20 µl of BD Multitest^{™} 6-color TBNK reagent (BD) and incubated for 20 min. at 4°C. After incubation, PBMCs were washed with PBS and cell pellets were resuspended in 1 ml of PBS containing 3 mM EDTA.PBMCs were filtered through 40 µm cell strainer to obtain real single cell suspensions by removing cell aggregates (clumps).

### 2.2. Isolation and labeling of white blood cells (WBCs) for sorting of granulocytes, monocytes and B lymphocytes

16 ml of whole blood were collected into two BD Vacutainer^{®} K2 EDTA tubes (BD). Red blood cells were lysed using 1× BD Pharm Lyse^{™} lysing solution (BD) added to blood samples up to 50 ml. Samples were incubated at room temperature for 10 min. The lysing step was repeated and then cells were then washed with PBS. Isolated white blood cells (WBCs) were stained with following antibodies: 5 µl of PE-labeled CD14, clone MϕP9 (BD) and 20 µl of APC-labeled CD19, clone HIB19 (BD) and incubated for 20 min. at 4°C. After incubation, WBCs were washed with PBS and cell pellets were resuspended in 2 ml of PBS containing 3 mM EDTA. WBCs were filtered through 40 µm cell strainer to obtain real single cell suspensions by removing cell aggregates (clumps).

### 2.3 Sorting of target cell populations with FACS

The target populations of cells were isolated using FACS Aria III (Becton Dickinson) at Uppsala University, FACS Aria II (Becton Dickinson) at Jagiellonian University Collegium Medicum or MoFlo (Beckman Coulter) at Jagiellonian University. Data was acquired and analyzed using BD FACSDiva^{™} Software (Becton Dickinson) with FACS Aria III and FACS Aria II or Summit^{™} Software System (Cytomation) with MoFlo. Compensation was determined using cells stained with single antibody from BD: FITC-labeled CD3, clone UCHT1; PE-labeled CD14, clone MϕP9; PerCP-Cy^{™}5.5-labeled CD45, clone HI30; PE-Cy^{™}7-labeled CD4, clone SK3; APC-labeled CD19, clone HIB19; APC-Cy7-labeled CD8, clone SK1.

Live cells were sorted based on their FSC and SSC. CD4+ T cells were defined as CD45+CD3+CD8-CD4+; CD8+ T cells were defined as CD45+CD3+CD4-CD8+; B cells were defined as CD45+CD3-CD19+; NK cells were defined as CD45+CD3-CD4-CD16+CD56+; monocytes were defined based on their size and as CD14+; granulocytes were defined based on their size and granularity and additional B cells were defined based on their size and as CD19+. Cells were sorted to achieve the purity of above 96%. At least 200 000 cells of each type were sorted. After sorting, cells were centrifuged for 5 min. at 400 RCF and 1 ml of RNAProtect (Qiagen) was added to cell pellets. Resuspended cells were centrifuged for 5 min. at 400 RCF, the supernatant was removed and the cell pellets were frozen immediately on dry ice for further processing.

### 3. Establishment of lymphoblastoid cell lines (LCLs)

Peripheral blood mononuclear cells (PBMC) were isolated from blood by Ficoll-Paque gradient separation. PBMC were infected with EBV by incubating them with supernatant1 of the virus producing B95-8 line for 90 min at 37 °C. Thereafter the cells were washed and resuspended in complete RPMI-1640 medium (supplemented with 10% heat inactivated FCS, penicillin and streptomycin). 1 µg/ml Cyclosporine A was added to the cultures in order to inhibit T cell expansion and function. After 3 weeks of culturing, transformed cells were plated into microtitre plates with U-shaped bottom at a 1 cell/well density. In each well, gamma-irradiated human fibroblasts (5000 rad) served as feeder cells. Growing clones were expanded and further analyzed. Clones showing either 100% LOY or 100% normal cells were selected for further study. The percentage of LOY was determined using both genotyping and ddPCR technologies (see below).

### 4. LOY analyses using DNA from sorted cells and LCLs

### 4.1 DNA extraction

DNA was extracted from cell pellets of sorted cells using an in-house protocol. The cell pellet was resuspended in lysis buffer containing 10 mM EDTA, 10 mM Tris-HCL (pH 7.9), 50 mM NaCl, 1% N-Lauroylsarcosine sodium salt (Sigma) with 10 mg/ml proteinase K (Sigma) and incubated for 2 hours in 50°C. The DNA was then precipitated using Sodium Acetate (pH 5.4) and 96% Ethanol, washed with 80% Ethanol and resuspended in MQ water. DNA was extracted from whole blood using QlAmp DNA Blood Midi kit (Qiagen) according to manufacturer's protocol. The concentration of DNA was measured using Quant-iT^{™} PicoGreen^{®} dsDNA Assay Kit (Thermo Fisher Scientific) according to manufacturer's instructions, and analyzed using plate reader Infinite M200 (Tecan Diagnostics). DNA was stored in -20°C freezer.

### 4.2 Genotyping using SNP-arrays

All genotyping experiments were performed following the manufacturer's instructions at the Science for Life technology platform SNP&SEQ at Uppsala University, Sweden. DNA extracted from the 2661 FACS sorted populations (collected from 408 subjects) was genotyped using three different versions of Illumina SNP-arrays (242 samples on the InfiniumCoreExome-24v1-1, 60 samples on the InfiniumOmniExpressExome-8v1-3 and 2359 samples on the InfiniumQCArray-24v1). The DNA extracted from the 13 LCLs was genotyped using the InfiniumQCArray-24v1.

### 4.3 Experiments using digital droplet PCR (ddPCR)

Additional quantification of the level of LOY for validation was performed using previously described procedure (Danielsson et al., 2019). Briefly, extracted DNA from the established LCL cell lines was digested for 15 min with Hindlll enzyme (Thermo Fischer) in 37 °C. After this, 50 ng of digested and diluted DNA was added together with PCR primers and probes targeting a known difference between the AMELX and AMEL Y gene assay C_990000001_10 (Thermo Fisher) to ddPCR supermix for probes no dUTP (Bio-Rad). Droplets were then generated using an automated droplet generator (Bio-Rad). Following this, the digested DNA was amplified using PCR. Droplets fluorescence intensity in two channels FAM and VIC was measured using the Bio-Rad's QX200 Droplet Reader.

### 5. Experiments using bulk RNA from sorted cells and LCLs 5.1 RNA extraction

RNA was extracted from cell pellets using RiboPure^{™} RNA Purification Kit (Thermo Fisher Scientific) according to the manufacturer's instructions. A possible DNA contamination was removed using TURBO DNA-free^{™} Kit (Thermo Fisher Scientific) following the manufacturer's protocol. The RNA was then concentrated using GeneJET RNA Cleanup and Concentration Micro Kit (Thermo Fisher Scientific) according to manufacturer's instructions. RNA quality was measured with RNA Pico Chip (Agilent Technologies) following manufacturer's protocol, and analyzed using Agilent 2100 Bioanalyzer (Agilent Technologies). RNA was kept on ice during handling and was stored in -80 ⁰C freezer.

### 5.2 Bulk RNA sequencing (RNAseq)

Bulk RNA sequencing was performed by the Science for Life technology platform at Uppsala Genome Centre (Uppsala University, Sweden). The applied method quantifies the amount of mRNA of different genes (about 20,000) by amplicon-specific primer pairs (usually targeting exon 1 and exon 2 of the spliced mRNA) in PCR amplification followed by next-generation sequencing. For each sample, an average of 10 ng of RNA (1 - 100 ng) from the 150 FACS sorted samples was used. Library preparation was performed using the Ion Ampliseq Human Gene Expression kit (Thermo Fisher Man0010742). Briefly, cDNA libraries were first constructed followed by amplification using Human Gene Expression Core panel (Thermo Fisher). For samples with a low amount of starting RNA the number of amplification cycles was increased (to 11 - 16 cycles, depending on RNA abundance in the specimen). Then, IonCode (Thermo Fisher) barcodes were ligated to the amplified mRNA. Amount of RNA in each library was estimated using a Fragment Analyzer instrument (Agilent). Libraries were loaded onto Ion 550 chips following recommendations from the manufacturer (MAN0017275) and sequenced using an Ion S5 XL instrument. In total, 51 monocyte and granulocyte samples and 48 NK cell samples were sequenced. Out of these samples, 2 granulocyte and 2 NK cell samples failed to produce sequencing results.

### 6. Experiments using RNA from single cells 6.1 Sample preparation

Whole blood was collected from Swedish men using BD Vacutainer CPT tubes (BD Biosciences) following the manufacturer's instructions and stored on ice. PBMCs were isolated using density gradient centrifugation and resuspended in 0.04% BSA 1X PBS solution. Concentrations were measured using an EVE cell counter (NanoEnTek, Seoul) and cells were diluted to 10⁶ cells/ml. The cells were then delivered to the Science for Life SNP&SEQ Technology platform at Uppsala University, Sweden.

### 6.2 Single cell RNA sequencing (scRNAseq)

The collected PBMCs were loaded on a 10X Genomics Chromium Single Cell 3' Chip v2 for sequence library generation using protocol CG00052 (10X Genomics). The applied method quantifies the amount of mRNA of different genes in single-cells using a 3'-end protocol. It allows for gene expression profiling of up to 10,000 individual cells per sample by construction of Gel Bead Emulsions (GEMs). Each GEM contains a single cell together with reagents such as (i) an Illumina R1 sequence (read 1 sequencing primer), (ii) a 16 nt 10x Barcode, (iii) a 10 nt Unique Molecular Identifier (UMI), and (iv) a poly-dT primer sequence. Incubation of the GEMs produces barcoded, full-length cDNA from poly-adenylated mRNA. GEMs are thereafter broken and the pooled fractions are recovered and sequenced. The above steps (including sampling and sample preparation) were performed within 5 hours. The single cell libraries were sequenced on Illumina HiSeq2500 and NovaSeq instruments, according to manufacturer's instructions. All single cell library preparation and sequencing were performed at the Science for Life technology platform SNP&SEQ, Uppsala University, Sweden.

### 7. Measurements of plasma protein abundance in men with LOY

To investigate if LOY leaves a footprint in the plasma proteome previously generated data from NSPHS cohort was analyzed. The fraction of cells with LOY was estimated from sequencing data and protein abundance in the same subjects was measured using Proximity Extension Assays (PEA).

### 7.1 Whole genome sequencing (WGS)

From all 480 men in the NSPHS cohort, 30X whole genome sequencing (WGS) was available for LOY analysis and generated as follows. Sample DNA was fragmented using a Covaris E220 (Covaris Inc., Woburn, MA, USA) to an insert size of 320 bp. Sequencing libraries were prepared using 1.1/1 *µ*g DNA using the TruSeq DNA PCR free sample preparation kit (illumina Inc. guide 15036187). Libraries were sequenced using an Illumina HiSeq X instrument (HiSeq Control Software 3.3.39/RTA 2.7.1, v2.5 sequencing chemistry). The sequencing was performed at the Science for Life SNP&SEQ technology platform, Uppsala University, Sweden.

### 7.2 Proximity Extension Assays (PEA)

From the same set of 480 NSPHS men, plasma protein abundance of 441 proteins was available. This data was generated using five different PEA-panels commercially available from Olink Proteomics, Uppsala, Sweden (www.olink.com). Specifically, panels CVD II, CVD III, INF I, ONC 2 and NEU I were analyzed by Olink company. The sample preparation and PEA workflow has been described previously (Enroth et al., 2018).

### QUANTIFICATION AND STATISTICAL ANALYSIS

### 8. Quantification of LOY from DNA

### 8.1 LOY analysis using SNP-array data

All included experiments passed strict quality control at the genotyping facility and in addition, we calculated for every experiment the sdLRR1, i.e. the standard deviation of Log R Ratio of probes on chromosome 1. Only samples with sdLRR1 <0.28 were considered to have good quality and included in downstream analyses, as recommended by Illumina

(Technical note from Illumina, https://www.illumina.com/content/dam/illuminamarketing/documents/products/appnotes/appn ote_cnv_loh.pdf). For each sample, we calculated the median of the log R ratio values of the SNP-array probes located in the male specific region of chromosome Y (MSY), i.e. the continuous mLRRY, as described previously (Forsberg et al., 2014). To correct for genotyping batch effects, we calculated the local regression median (LRM) in each batch and the mLRRY value for each subject was thereafter corrected by the batch-specific LRM. The percentage of normal cells without LOY in each sample was estimated using a formula described previously, i.e. 100*(2^{2*mLRRY}) (Danielsson et al., 2019).

### 8.2 LOY analysis using ddPCR data

The data generated by the Bio-Rad's QX200 Droplet Digital PCR System was analyzed in Bio-Rad's software QuantaSoft (version 1.7.4.0917) as described elsewhere (Danielsson et al., 2019). Briefly, the amount of DNA of *AMELY* was divided by the amount of DNA for *AMELX* in each sample. The ratio represents an unbiased estimate of the level of LOY in the examined samples.

### 8.3 LOY analysis from WGS data

We used for scoring of LOY from whole genome sequencing data, a pipeline similar to previously described (Dumanski et al., 2016). Briefly, sequencing raw reads were mapped and aligned using BWA-MEM 0.7.12 and v. GRCh37 of the human reference, followed by sorting and indexing by Samtools 0.1.19. Alignments from different lanes and flow cells were merged using Picard (1.120). In order to estimate the amount of LOY in each male sample the software ControlFREEC (version *FREEC-11.5*) (Boeva et al., 2011) was used. ControlFREEC calculates read depth ratios for genomic windows from WGS-data while taking mappability for each region into account. As the ratio produced by ControlFREEC will equal to 1 for any region with two chromosomes and without any traces of CNVs, a Y chromosome region with the same conditions for a male subject without LOY will be 0.5. The Y-chromosome ratio was therefore multiplied by 2 for each male in order to get a Y chromosome ratio between 0 and 1. For the ControlFREEC settings, it was given the same references file that was used to create the bam-files as well as the 2 mismatches, hg19 and read length of 100 base pairs mappability file linked in programs manual (ControlFREEC v11.5). The settings were kept as default, except for the window-size set to 50.000 base pairs.

### 9. Quantification of LOY from RNA

### 9.1 Analysis pipeline of bulk RNAseq data from sorted cells

For each of the FACS sorted samples analyzed using the Ion Ampliseq Human Gene Expression kit, raw read-counts were read into the R (v3.5.3) software, and one matrix of read-counts was created per cell type. Outliers were searched for within each cell type using principal component plots. Only among the monocyte samples, eight outliers were identified and removed. To remove low quality expression data, the count matrices were filtered by creating two groups of samples (i.e. more and less than 50% LOY). In each group, only genes with at least 10 reads in one third of the samples were used.

In order to identify the normally expressed genes in the sorted cell fractions, we focused on samples without LOY. In these, a minimum of 5 reads in at least one third of the samples was required to be considered a normally expressed gene. In order to estimate the level of LOY in each sample in the RNAseq data, a variance-stabilizing transformation was first applied (DESeq2, varianceStabilizingTransformation function) to the count matrix in order to make the expression more coherent. A mean expression was then calculated for all genes on the autosomal chromosomes, and compared to the mean expression of the MSY-genes, generating a fraction of expression for the Y-chromosome. The calculated fractions were rescaled to fit between 0 and 100. This was done per cell-type, to remove cell type specific effects on expression levels.

We estimated the LOY associated transcriptional effects (LATE) as follows. The R library DESeq2 (v1.22.2) was used to identify genes with differential expression in the leukocytes derived from blood for each patient. As LOY is a continuous trait, the percentage of LOY estimated from SNP-arrays for each sample was used for statistical testing. To decrease the impact of read-count outliers the fraction of LOY was binned using 20% intervals between <20% - 80%< and these bins were used as a continuous input variable for DESeq2 (Love et al., 2014). To avoid batch effects batches were introduced as a covariate in the analysis. Independent filtering of genes in DESeq2 was done using 0.1 as a value for the alpha parameter, which was also used as a cutoff for the DESeq2 adjusted p-values.

To investigate the relation between LOY and expression level of MSY genes, we applied a non-linear least square model, using nls() function in R, on average expression of all selected MSY genes (6 in panel A and 13 in panel B) and percentage of LOY in samples of each cell type. For each cell type, the expression level of each sample has been divided by the maximum expression level among all samples in order to keep all values in the same scale of 0 to 1.

### 9.2 Analysis pipeline of bulk RNAseq data from LCLs

We used R (version 3.5.2) and the edgeR (version 3.24.3) library to compare the expression between LOY and non-LOY group of samples in two steps. As all the clones with LOY were derived from the same individual (this individual also gave rise to one non-LOY clone) we designed the analysis to remove inter and intra individual differential expression effects.

As step one, in order to identify the intra-individual differences, we targeted the differential expression genes among all non-LOY clones by comparing each non-LOY individual's clones with all other non-LOY clones. The union of all differentially expressed genes resulted out of each comparison was gathered into List1 (**Figure 11****).** This gave us a set of genes that are differentially expressed between subjects but not due to LOY status. As step two, we targeted the differentially expressed genes regarding LOY status in two steps: first by comparing the non-LOY versus LOY clones derived from the same individual; second by comparing all non-LOY versus LOY clones and the overlap of the results of steps one and two was gathered into List2. The final list of LATE genes was generated by comparing List2 and List1 and extracting the differentially expressed genes that exist in List2 but not in List1, to remove the intra-individual differentially expressed genes from the LATE genes.

### 9.3 Analysis pipeline of single cell scRNAseq data

Sequence reads were mapped to the hg19 version of the human genome using Cell Ranger v2 (10X Genomics) using recommended settings. The produced raw reads matrices were analyzed using R (v3.4) and the R library Seurat (v2.2) (Butler et al., 2018). For each sample the read count matrix was used to create a Seurat object in R. After this all data was log normalized and variable genes were identified using standard settings from the Seurat manual. Data was then scaled and the principal components were calculated. Then, the number of informative principle components for each sample was identified using an elbow plot and cells were then clustered using the findclusters Seurat function and informative principle components.

A tSNE plot was produced with the resulting clusters of the nearest neighbor clustering indicated by the color of each cell in the tSNE plot, to confirm consistency between the two methods. An in house R script was then used to calculate the level of expression of cell marker genes in each cluster and cell types was assigned using the strongest expression signal. These results were then manually inspected using heatmaps in order to confirm the automated cell-type assignment.

For each sequenced cell, the number of reads mapping to genes in the MSY was counted. Cells having a LOY transcriptional profile were identified as cells having no expression from genes in the MSY. To calculate percentage of LOY in each sample and cell-type the fraction of cells having LOY was calculated for each specific cell type. Following this step, all individuals were merged together creating one object for each studied cell type (monocytes, NK cells, T- and B lymphocytes). The procedure outlined above was repeated for the merged data-sets and the cells were filtered only keeping cells with at least 800 expressed genes but no more than 2000 expressed genes. This was done both to remove experiments where two cells were sequenced together, and to remove cells with a generally low expression of genes, where the LOY estimate may be erroneous. Cells with more than 5% mitochondrial RNA were also excluded, in order to remove possibly apoptotic and damaged cells from the analysis.

Normally expressed genes in the scRNAseq data were defined by the expression in at least 10% of single cells without LOY. After establishing the normally expressed genes in each cell type in the scRNAseq data, we further filtered the cells to only include only cells having more than 2500 UMIs. This was done in order to remove possible false positive LOY cells, and only keep cells where a robust and high number of UMIs was found. We then identified LATE genes using the Seurat FindMarkers function. We used this function to test differences in gene expression in cells with and without LOY using the built-in negative binomial test.

### 9.4 Co-expression analysis

We investigated if changes in expression as an effect of LOY would be larger in autosomal genes that are normally co-expressed with MSY genes, compared with genes that are not co-expressed with MSY genes. Six MSY genes that are normally expressed in lymphocytes were used in this analysis (i.e. *RPS4Y1, ZFY, USP9Y, DDX3Y, KDM5D* and *EIF1AY*). Genes normally co-expressed with these MSY-genes were identified using the SEEK database [http://seek.princeton.edu/]. After this we calculated the fold change of the expression between LOY and normal cells for each cell-type. We then selected the top 300 co-expressed genes retrieved from the SEEK database and for each of these genes we identified if they were expressed in our scRNAseq data or not. Following this the fold changes for genes found to be co-expressed and genes that were not co-expressed was used to produce plots and p-values (Wilcoxon rank sum test).

### 10. Gene ontology (GO) functional annotation

For annotation of known functions of chromosome Y genes for **Fig. 1**, a list of all genes located on the Y chromosome together with associated GO categories was downloaded from Ensembl (v. 95) using the R library bioMart (v2.38). The list was filtered for protein coding genes and annotated as either positioned in the male specific part of the Y chromosome (MSY) or in the pseudo autosomal regions (PAR). The GO categories for each gene were inspected manually and genes were assigned into four different categories: *"Transcription, translation epigenetic and PTM functions", "Cell differentiation, migration proliferation and apoptosis", "Sex determination, fertility and other functions", "Immune response and immune cell signaling".*

We also performed GO analyses of the LATE genes identified using a stringency level of p<0.05 (n=3360) as well as for LATE genes showing differential expression using the more stringent significance level of FDR<0.1 (n=521) in the samples studied *in vivo* using scRNAseq and RNAseq. The analyses were performed using GO Ontology database (http://geneontology.org/) on the 2019-05-24 using the "GO *biological process complete"* option, the Fisher's exact test, and the FDR correction method. As input we used the 521 and 3360 LATE genes showing differential expression as an effect of LOY and as reference background, we used all the expressed genes identified *in vivo* in the scRNAseq and RNAseq experiments (n=11859).

### 11. Analysis of protein abundance

Analysis of the protein data was performed using an adapted Personally Normalized Plasma Protein Profiles (PNPPP) method (Enroth et al., 2015). Briefly, variance in protein abundance levels not related to LOY was accounted for by first examining a set of 159 covariates (anthropometrics, lifestyle variables, medication and genetic markers) using ANOVA in individuals not affected by LOY. The genetic marker was defined as the dosages for the top-hit for each protein as reported in Enroth et al 2018 (Enroth et al., 2018). Age was always included as a covariate due to the high correlation between LOY and age. The models generated using the individuals not affected by LOY were then used to adjust the protein abundance levels in all individuals, including those affected by LOY. In a second step, adjusted protein levels were compared between the two groups and a two-sided Spearman's ranked test was used to calculate p-values. A Bonferroni adjusted q-value of 0.05 was used to determine statistically significant differences. Out of the 441 proteins 16 had <20% of samples above the limit of detection and were excluded from the final analysis.

### 12. Statistical analyses

Statistical analyses such as logistic regression, linear regression, Fisher exact test, Kolmogorov-Smirnov test and Wilcoxon rank sum test was performed using R version 3.3.1 (http://www.rproject.org/), using two-sided tests and α-levels at 0.05 and default parameters, unless otherwise specified in the text. When appropriate, correction of p-values for multiple testing was applied, such as Bonferroni correction in the protein analyses (**Figure 7**). In order to define the LATE genes, we applied three stringency levels. The first was based on standard p-value at α-level 0.05 and the second was a more strict p-value correcting for multiple testing (FDR<0.1) using Benjamini and Hochberg (BH) correction (**Table 1**). Furthermore, an additional criterion was applied to identify only the highly expressed LATE genes, i.e. using threshold of at least an average of 100 reads per gene in RNAseq data (**Figure 6**).

### REFERENCES FOR METHODS

Assarsson, E., and Lundberg, M. (2017). Development and validation of customized PEA biomarker panels with clinical utility. IN: Advancing precision medicine: Current and future proteogenomic strategies for biomarker discovery and development (Washington, DC: Science/AAAS), pp. 32-36.
Boeva, V., Zinovyev, A., Bleakley, K., Vert, J.P., Janoueix-Lerosey, I., Delattre, O., and Barillot, E. (2011). Control-free calling of copy number alterations in deep-sequencing data using GC-content normalization. Bioinformatics 27, 268-269.
Butler, A., Hoffman, P., Smibert, P., Papalexi, E., and Satija, R. (2018). Integrating single-cell transcriptomic data across different conditions, technologies, and species. Nat Biotechnol 36, 411-420.
Danielsson, M., Halvardson, J., Davies, H., Torabi Moghadam, B., Mattisson, J., Rychlicka-Buniowska, E., Heintz, J., Lannfelt, L., Giedraitis, V., Ingelsson, M., et al. (2019). Intra-individual changes in the frequency of mosaic loss of chromosome Y over time estimated with a new method. submitted.
Dumanski, J.P., Lambert, J.C., Rasi, C., Giedraitis, V., Davies, H., Grenier-Boley, B., Lindgren, C.M., Campion, D., Dufouil, C., European Alzheimer's Disease Initiative, I., et al. (2016). Mosaic Loss of Chromosome Y in Blood Is Associated with Alzheimer Disease. Am J Hum Genet 98, 1208-1219.
Enroth, S., Bosdotter Enroth, S., Johansson, A., and Gyllensten, U. (2015). Effect of genetic and environmental factors on protein biomarkers for common non-communicable disease and use of personally normalized plasma protein profiles (PNPPP). Biomarkers 20, 355-364.
Enroth, S., Maturi, V., Berggrund, M., Enroth, S.B., Moustakas, A., Johansson, A., and Gyllensten, U. (2018). Systemic and specific effects of antihypertensive and lipid-lowering medication on plasma protein biomarkers for cardiovascular diseases. Sci Rep 8, 5531.
Forsberg, L.A., Rasi, C., Malmqvist, N., Davies, H., Pasupulati, S., Pakalapati, G., Sandgren, J., de Stahl, T.D., Zaghlool, A., Giedraitis, V., et al. (2014). Mosaic loss of chromosome Y in peripheral blood is associated with shorter survival and higher risk of cancer. Nature Genetics 46, 624-628.
Love, M.I., Huber, W., and Anders, S. (2014). Moderated estimation of fold change and dispersion for RNA-seq data with DESeq2. Genome Biol 15, 550.

### RESULTS AND DISCUSSION

### Different distribution of LOY in six types of sorted leukocytes among men with AD and PC

Studies of associations between LOY and various outcomes have until now focused on analyses of DNA from peripheral blood. Our investigation of a possible causality behind associations between LOY in blood and risk for diseases in other organs started with investigation of LOY in six major types of leukocytes sorted with a Fluorescent Activated Cell Sorter, in patients with AD, PC and healthy age-matched controls. Our working hypothesis was that there might be a difference in LOY frequency in various types of leukocytes between patients with AD and PC. The six subsets of sorted leukocytes were: CD19+ B lymphocytes; CD4+ T lymphocytes; CD8+ T-cytotoxic lymphocytes; Natural Killer (NK) cells; granulocytes; and monocytes. In total, we sorted leukocytes from 408 subjects; 228 patients with diagnosis of AD or PC and 180 healthy controls. We assessed the level of LOY from DNA in each subset using the most established method, i.e. SNP-arrays (Dumanski et al., 2016; Dumanski et al., 2015; Forsberg, 2017; Forsberg et al., 2014). The AD analysis included 215 subjects (121 patients and 94 age-matched controls) that were ≥70 years of age and free from cancer. The examination of PC included 263 subjects; 107 patients and 156 age-matched controls that were ≤80 years of age and free from AD (**Fig**. **2**). Two general conclusions could be drawn from comparisons of the level of LOY in each cell type in patients and controls. One is the overall trend that the degree of LOY mosaicism was higher in patients in all studied cell types, as shown in 12 pairwise comparisons from patients with AD and PC versus controls (**Fig. 2**). This is well in line with previous studies using whole blood DNA. The overall level of LOY, however, varied substantially between cell types with highest frequencies in the myeloid lineage and considerably lower levels in lymphoid cells (**Fig. 2**). The frequency of LOY in six cell types using four different cut-offs for LOY scoring is shown in **Fig. 10****.** The second general conclusion is that LOY was not equally distributed between different subsets of leukocytes in patients with AD or PC vs controls. It has been discussed whether the associations between LOY in leukocytes with various diseases and its strong association with increasing age, could merely reflect a stochastic age-dependent process of accumulation of various mutations in all cells (Forsberg et al., 2017). Our above results cells contradict this hypothesis.

We first used unadjusted logistic regression models with the continous mLRRY as response variable (Dumanski et al., 2016; Forsberg et al., 2014), which showed that men diagnosed with AD had more LOY in NK cells compared with controls (*p*=0.0109, **Fig. 2A**) and that men diagnosed with PC had more LOY in CD4+ T lymphocytes (*p*=0.0312, **Fig. 2D**). This result was confirmed by logistic regression models fitting the effects from age and smoking which are known LOY-associated confounders (Fig. 2B and E). Remarkably, in NK cells, LOY was 4.4 times more common in AD patients than in controls, using a cut-off representing ≥40% of NK cells with aneuploidy (**Fig. 9**). This could be compared to previous study of AD and LOY scored in whole blood DNA, showing an adjusted hazard ratio of 5.3 for AD-free follow up (Dumanski et al., 2016). Furthermore, in PC patients we observed that CD4+ T-lymphocytes were more commonly affected with LOY (**Fig**. **8**). However, the level of LOY in this sorted subset was considerably lower than in NK cells from AD patients. This might reflect that CD4+ lymphocytes is a heterogeneous group of cells and a possibility that LOY in only a fraction of these cells is connected with an increased risk for disease in PC patients. Consequently, further studies should focus on specific subsets within CD4+ lymphocytes.

NK cells are an important part of the host defense and the immune system has been suggested as severely disturbed in AD (Larbi et al., 2009; Speciale et al., 2007). Furthermore, NK cells have been proposed as involved in the pathogenesis of AD in studies performed in humans and animal models (Araga et al., 1991; Jadidi-Niaragh et al., 2012; Korin et al., 2017; Le Page et al., 2015; Le Page et al., 2018; Masera et al., 2002; Mrdjen et al., 2018; Schindowski et al., 2006; Solerte et al., 2000). However, the role of NK cells in AD has not been sufficiently explored, especially when the accumulation of post-zygotic mutations acquired during lifetime (such as LOY) is considered. The presented results support the possibility that LOY in certain types of leukocytes contributes to increased risk for specific diseases. Furthermore, the investigations of LOY in the context of associations with various diseases have until now typically used whole blood DNA. Overall, our results suggest that this strategy should be revised and we ought to consider using in future LOY studies DNA from the subsets of leukocytes that are most relevant for the disease under investigation. In conclusion, we provide the first indication that LOY in NK cells and CD4+ lymphocytes might contribute to the pathogenesis of AD and cancer, respectively, presumably by impairment of normal immune system functions.

Our results also suggested that cell clones with LOY often might be a result of oligo-clonal cell expansions in peripheral blood. In Figure 2C and F, we show data for oligoclonality of LOY (ocLOY) in men with a diagnosis of AD or PC vs controls. OcLOY was defined as having LOY in ≥20% of cells in at least two of the six types of sorted leukocytes. We identified 52 subjects according to this definition and found that a majority of men with ocLOY had diagnosis of AD or PC. In the AD cohort, ocLOY was significantly higher in patients (OR=2.64, *p*=0.0127) with 24.0% of patients (median age=80, range=70-95) compared to 10.6% of controls (median age=75, range=70-95). A comparable difference was observed in the PC cohort in which 9.3% of the patients (median age=66, range=51-80) displayed compared to 5.1% of controls (median age=69, range=45-79). An overall analysis combining all AD and PC patients showed a significantly higher level of ocLOY compared to controls (OR=2.74, *p*=0.0026). Since the median age was slightly higher in patients than controls, we also investigated the frequency of ocLOY in a subset of subjects between 70 and 80 years of age. This analysis confirmed that OcLOY was more common (OR=2.87, *p*=0.0462) in patients (N=110, 18.2% with OcLOY, median age=76, range=70-80) than controls (N=70, 7.1% with OcLOY, median age=74, range=70-79). These results may suggest that the overall load of LOY in leukocytes may also contribute to disease development, in addition to involvement of specific cellular subsets with LOY in patients with AD and PC (i.e. NK cells and CD4+ T-lymphocytes, respectively).

### The frequency of cells with LOY can be estimated from mRNA transcriptome

Our next goal was to investigate mRNA expression in samples of sorted cells and single-cells as well as in cells *in vitro.* The first step was to estimate the level of LOY from mRNA datasets. We studied transcriptome using two independent technologies, namely single cell RNA sequencing (scRNAseq) of peripheral blood mononuclear cells (PBMCs) and sequencing of bulk RNA (RNAseq) from the sorted leukocytes and 13 lymphoblastoid cell lines (LCLs). The latter were derived from single cell clones and encompassed five LCLs with 100% LOY cells as well as eight LCLs containing essentially 100% of cells with chromosome Y (see below).

For scRNAseq results, each sequenced cell with expression of autosomal genes, but without any transcripts from genes located in the male specific region of chromosome Y (MSY) was considered as a LOY cell. The single cell analyses of PBMCs collected from 29 men (26 diagnosed with AD) generated a scRNAseq dataset encompassing 73,606 cells analyzed using established algorithms, as described in methods and elsewhere (Thompson et al., 2019). The distribution and level of LOY estimated from scRNAseq in four major cell types (i.e. NK cells, monocytes, B- and T lymphocytes) is shown in Figure 3A and B. The level of LOY in NK cells, monocytes, B- and T lymphocytes in the pooled scRNAseq dataset were 27% (range 7-87%), 23% (range 7-87%), 7% (range 2-40%) and 3% (range 1-6%), respectively. Noteworthy, we identified single cells with LOY in all subjects studied by scRNAseq. Concerning bulk RNAseq data, the level of LOY was estimated by comparing the stabilized mean number of mRNA transcripts from MSY genes with the corresponding values of autosomal genes. The resulting ratios were rescaled to fit between 0 and 100, to produce estimates of LOY. This analysis was performed for 134 samples (NK cells, monocytes and granulocytes) derived from cell sorting of 51 subjects (Fig. 3C and D).

In order to evaluate the mRNA-based LOY estimates, we performed pairwise comparisons for scRNAseq and RNAseq against the DNA-based LOY scoring, using the same set of samples (**Fig**. **4**). We also compared the two mRNA-based methods and the Pearson's correlation coefficients for these pairwise analyses were 0.93, 0.92 and 0.91, respectively. This shows that the performance of LOY estimations from scRNAseq and RNAseq is reliable. Concerning the 13 LCLs, LOY estimation was performed using two methods; array-based SNP genotyping and droplet digital PCR (ddPCR); the latter utilizing a common polymorphism within amelogenin gene located on chromosomes X and Y (Sullivan et al., 1993). This ddPCR-assay is a recent methodological development allowing rapid scoring of LOY and has been described elsewhere (Danielsson et al., 2019). The estimations of the level of LOY from both technologies were highly concordant with Pearson's correlation coefficient of 0.9961.

### LOY causes a reduced level of expression of genes from chromosome Y

The study of **L**OY **A**ssociated **T**ranscriptional **E**ffect (LATE) started with analyses of the expression of genes located on chromosome Y. According to Ensembl (v. 95), the Y chromosome contains 64 protein coding genes: 45 in MSY and 19 in the pseudo-autosomal regions (PARs). We detected in total expression of 20 protein coding genes from chromosome Y in leukocytes studied with RNAseq and scRNAseq (**Fig. 1**). The determination of normally expressed genes from chromosome Y was based on observations in samples and cells without LOY. Hence, in the bulk RNAseq dataset, genes with at least 10 sequencing reads in more than 75% of the samples without LOY were considered normally expressed. For the scRNAseq data, expression in at least 10% of single cells without LOY was required to be defined as a normally expressed gene. Of the 20 protein coding genes located on chromosome Y and showing expression using above criteria, seven are located in the MSY and 13 in the PARs.

After establishing which MSY genes were normally expressed in the studied cell subsets, we investigated the level of expression of these genes in relation to the level of LOY in each sample. The analyses of expression of MSY genes were performed with the dataset generated with bulk RNAseq from sorted cells. Similar analyses with the scRNAseq data could not be performed, since single cells with LOY are per definition lacking transcripts from MSY genes. The analyses of the bulk RNAseq dataset showed that the normalized abundance of transcripts from the MSY genes clearly decreased with increasing level of LOY (**Fig. 5A**). A corresponding analysis of the genes located in the PAR also showed a decrease in transcript abundance with increasing levels of LOY. However, this LOY associated decrease was not as distinct as for the MSY genes, which can be explained by sustained expression of the chromosome X-copy of PAR genes (**Fig. 5B**). . The effect on gene expression from a continuous LOY estimate was quantified using an established algorithm calculating the size and direction of the differential expression (DE) using regression analysis (Love et al., 2014). DE-values close to zero suggest no change and positive and negative DE-values indicate over- and under-expression, respectively. Among the PAR genes showing a lower expression as an effect of LOY is the CD99 gene **(**Fig. 6B and D). Its product is a cell surface glycoprotein involved in processes such as leukocyte migration, cell adhesion and apoptosis; e.g. by functioning as a diapedesis-mediating receptor central for migration of monocytes through endothelial junctions (Schenkel et al., 2002; Vestweber, 2015). A lower expression of *CD99* in leukocytes with LOY might therefore mitigate extravasation and thus impair the recruitment and movement of leukocytes from circulation towards somatic tissues and sites of disease. In summary, samples with LOY showed a lower abundance of mRNA transcripts from genes located in MSY and PARs. This provides proof of principle and serve as an internal control that LATE can be confidently estimated by transcriptome analyses.

### Effects beyond the Y chromosome; autosomal LATE genes

In order to investigate whether LOY causes transcriptional effects of genes located on other chromosomes, we first determined the autosomal genes that are normally expressed in each studied cell type and subsequently measured their expression level in relation to LOY. The criteria used to identify normally expressed autosomal genes were the same as for the analyses of genes located on chromosome Y (see above). To determine differential expression of these genes as an effect of LOY we used two approaches: for the dataset generated with RNAseq we used the DESeq2 package in R (Love et al., 2014) and for the scRNAseq dataset, negative binomial testing was applied as implemented in the R library Seurat (Butler et al., 2018). Panel A in **Table 1** displays the number of normally expressed genes as well as the number of LATE genes (using two stringency levels) in the different cell types studied by the two mRNA-based technologies. In **Figure 6****,** an additional criterion was applied to identify only the highly expressed LATE genes.

Two types of global LATE analyses were performed and each showed that the overall autosomal gene expression might be affected by LOY. The first was LATE study of *in vitro* cultured LCLs with and without LOY (**Fig. 5** **C**). The LCLs were generated using Epstein-Barr virus transformation of lymphocytes collected from male donors with LOY in whole blood. DNA array as well as validation experiments using ddPCR were performed on viable LCLs and identified five monoclonal LCLs with 100% LOY cells as well as eight LCLs in which all cells carried chromosome Y. RNAseq of these 13 LCLs was performed and expression was investigated by principal components analysis. The first two principal components explained 45% and 19% of the total variance and a Kolmogorov-Smirnov test, comparing the variance in PC1 for LCLs with and without LOY, showed that the gene expression was affected in LCLs with LOY (D=1.0, p=0.0016). However, these results should be interpreted with caution since all LCLs with LOY in this analysis originated from the same donor (**Fig. 5C** **and** **Fig. 11**). It is nonetheless interesting that LCL1_2 clustered closer to the LCLs without LOY originating from other subjects, than to the other LCLs (with LOY) from this donor.

The second analysis of genome-wide LATE was performed using the dataset generated by scRNAseq from PBMCs collected *in vivo* (**Fig. 5D**). The hypothesis was that changes in expression as an effect of LOY would be larger in autosomal genes that are normally co-expressed with MSY genes, compared with genes that are not co-expressed with MSY genes. We mined the SEEK database for autosomal genes normally expressed in leukocytes and showing co-expression with six MSY genes expressed in all types of studied leukocytes (**Fig. 1**, **Fig. 5A**) and identified 275 co-expressed genes and 12852 control genes, i.e. without evidence of co-expression. The results showed a significantly larger dysregulation of the co-expressed genes, compared with control genes (Wilcoxon rank sum test: p=0.0021). Similar analyses were also performed separately within monocytes, NK cells, T- and B lymphocytes, in which 142, 116, 95 and 122 co-expressed autosomal genes as well as 3677, 3230, 2752 and 3075 autosomal genes without co-expression were identified, respectively. This showed that the global LATE was strongest in monocytes and T lymphocytes and weaker in NK cells and not detectable in B lymphocytes (**Fig. 5D**).

Hence, both types of global LATE comparisons described above supported an overall effect from LOY on expression of autosomal genes and the next step was investigation of specific autosomal LATE genes. In total, we identified 4026 LATE genes showing varying degree of differential expression in sorted leukocytes and LCLs studied by RNAseq as well as in PBMCs using scRNAseq. The analyses of cells *in vivo* identified 3366 LATE genes (3110 by RNAseq and 337 by scRNAseq) and 904 LATE genes by RNAseq in the LCLs (**Table 1A**). A clear difference between bulk RNAseq and scRNAseq is that expression of about 3 times more genes could be detected with the former method. The main reasons for this difference might be because a gene is not expressed in a particular cell at the time of sequencing or a failure to be sequenced due to technological limitations. For example, scRNAseq has lower depth of sequencing and measures only transcripts tagged with polyA-tail. In contrast, in bulk RNA sequencing, a much larger number of cells are studied simultaneously, yielding transcript reads also from genes with lower normal expression and from genes with temporal variation in expression.

Comparing the number of LATE genes within cell types with the fraction of LATE genes shared between cell types provide interesting insights regarding possible pleiotropic effects of LOY (**Tables 1B** and **3**). Pleiotropy can be defined as an effect from one feature at the genetic level to multiple characteristics at the phenotypic level (Paaby and Rockman, 2013). Both in the bulk RNAseq and in the scRNAseq analyses, the fraction of LATE genes was substantially larger within specific cell types than the fraction of LATE genes shared between different subsets (Kruskal-Wallis Chi-squared= 6.5, p=0.0105). For instance, up to 15% of normally expressed genes showed LATE in NK cells, but only about 2% are shared between NK vs granulocytes and NK vs monocytes. Moreover, the fraction of shared LATE genes was larger among the *in vivo* studied cells compared with the LCLs (Kruskal-Wallis Chi-squared= 3.9, *p*=0.0495). These results suggest that LOY may preferentially cause expression differences of autosomal LATE genes in a cell type specific manner. This pleiotropic effect can to some extent be explained by the fact that different cell types normally express distinct sets of genes. Hence, future studies of LATE should be performed within specific cell types, rather than less informative studies on samples of mixed leukocytes. However, we also identified many LATE genes that were dysregulated in more than one cell type. For example, among the 3110 genes with LATE identified in the RNAseq data originating from the *in vivo* collection, 338 LATE genes were detected in two cells types and 31 were shared between all three types of studied leukocytes. Similarly, for the 337 LATE genes identified by scRNAseq, 25 genes showed LATE in more than one cell type and two of these LATE genes were shared between all four cell types studied.

To investigate the biological functions of the identified LATE genes we first performed Gene Ontology (GO) analyses of the unique 3360 LATE genes discovered *in vivo* using a stringency level of p<0.05 (**Table 1**). We found these genes to be significantly enriched in 7 GO categories directly linked to immune system functions. For example, of the 3360 analyzed LATE genes we found 617 genes supporting the GO category *immune system process* and 423 supporting *immune response.* We also performed a corresponding GO analysis including only the 521 LATE genes showing differential expression using a more stringent threshold (i.e. FDR<0.1, **Table 1**) and this analysis validated the link between LATE genes and central immune system functions. Furthermore, for a subset of these LATE genes, we reviewed the literature for known functions and found many of them to have connections with the immune system and/or development of AD and/or cancer. A brief summary of relevant LATE genes is provided in **Table 4,** and among these genes are: *LAG3, LY6E, ANXA1, CSF1R, APBA3, CSF2RA, JAK3, IL15, CD3D, CD3G, CADM1, PYCARD, LILRB2, LILRA 1, KLRG1, HLA-DRA* and CCL5. One of the LATE genes that showed the strongest downregulation was the autosomal *LAG3* (Lymphocyte-activation gene 3) in NK cells (**Fig. 6**). The LAG3 protein is a cell surface molecule that functions as an immune checkpoint receptor by binding its main ligand MHC class II with higher affinity than CD4. Cellular proliferation and immune cell activation is regulated by a balance between CD4/LAG3, in a similar fashion to the CTLA4 and PD1 immune checkpoints, where *LAG3* expression suppresses cell activity (Huang et al., 2004; Workman et al., 2004). The observed low expression of *LAG3* in LOY cells might disrupt the CD4/LAG3 balance, which is noteworthy in the context of immune-surveillance and the increased risk for cancer observed in men with LOY.

In the NK cells and monocytes, studied by both RNAseq and scRNAseq, we identified 206 and 60 genes, respectively, showing high expression and profound LATE in RNAseq (**Fig. 6**). Furthermore, 18 genes in NK cells and 9 genes in monocytes were supported by both technologies. Among these, a handful were detected as LATE genes in both cell types; such as the PAR gene *CD99*, the MSY genes *EIF1AY* and *RPS4Y1,* as well as the autosomal gene *LY6E.* The *LY6E* gene (lymphocyte antigen 6 family member E) is clearly upregulated in expression studies (**Fig. 6**) and it has the potential to inhibit inflammatory cytokines and disrupt inflammatory cascades. A survey of more than 130 published clinical studies found that increased expression of *LY6E* is associated with poor survival outcome in multiple malignancies (Luo et al., 2016) and it has also been found to be important for drug resistance and tumor immune escape in breast cancer (AlHossiny et al., 2016).

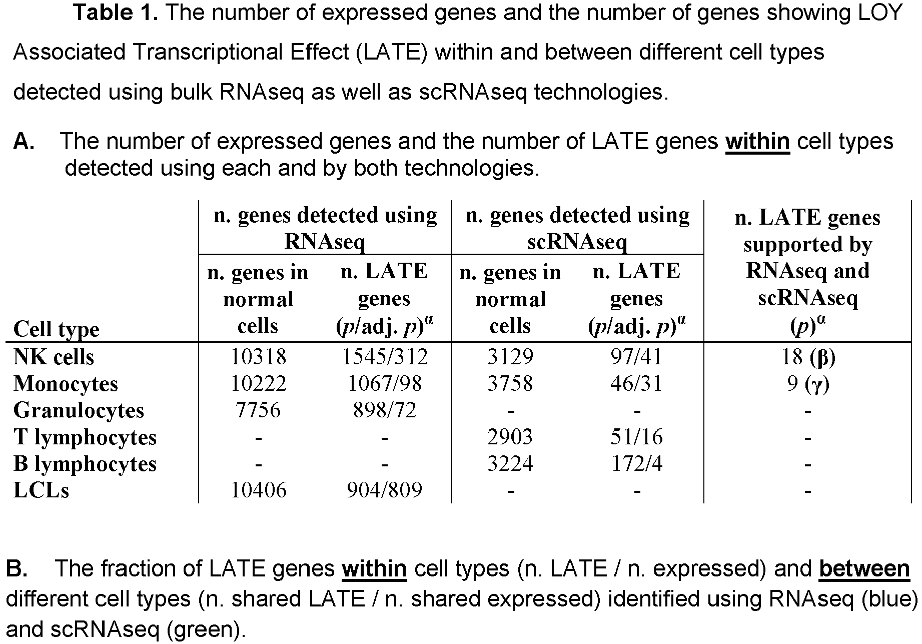

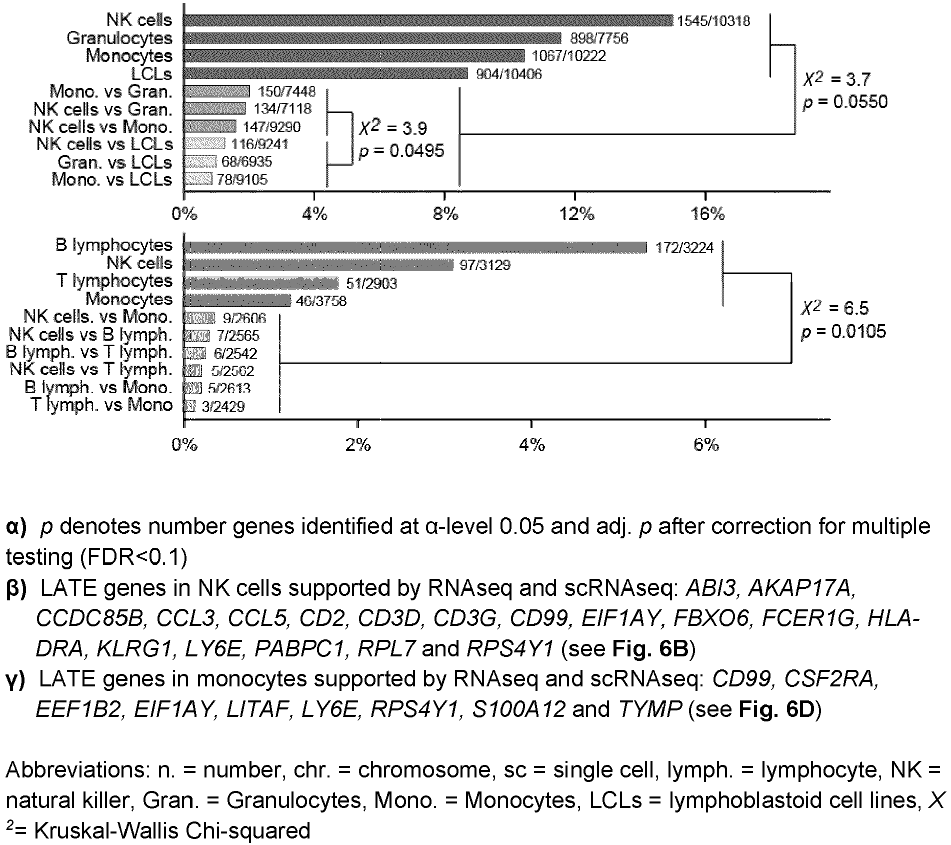

**Table 3. Related to Table 1.**

| **A) Number of expressed and LATE genes within cell types using RNAseq** | | | |
|---|---|---|---|
| **Cell type** | **# expressed genes (RNAseq)** | **# LATE genes (RNAseq)** | **Fraction** LATE **genes (%)** |
| **NK cells** | 10318 | 1545 | 14,97 |
| **Gran.** | 7756 | 898 | 11,58 |
| **Mono.** | 10222 | 1067 | 10,44 |
| **LCLs** | 10406 | 904 | 9,03 |

| **B) Number of shared expressed and LATE genes between cell types using RNAseq** | | | |
|---|---|---|---|
| **Cell type** | **# shared expressed genes (RNAseq)** | **# shared LATE genes (RNAseq)** | **Fraction shared LATE genes (%)** |
| **Mono. vs Gran.** | 7448 | 150 | 2,01 |
| **NK cells vs Gran.** | 7118 | 134 | 1,88 |
| **NK cells vs Mono.** | 9290 | 147 | 1,58 |
| **NK cells vs LCLs** | 9241 | 116 | 1,26 |
| **Gran. vs LCLs** | 6935 | 68 | 0,98 |
| **Mono. vs LCLs** | 9105 | 78 | 0,86 |

| **C) Number of expressed and LATE genes within cell types using scRNAseq** | | | |
|---|---|---|---|
| **Cell type** | **# expressed genes (scRNAseq)** | **# LATE genes (scRNAseq)** | **Fraction LATE genes (%)** |
| **B Lymph.** | 3224 | 172 | 5,33 |
| **NK cells** | 3129 | 97 | 3,10 |
| **T Lymph.** | 2903 | 51 | 1,76 |
| **Mono.** | 3758 | 46 | 1,22 |

| **D) Number of shared expressed and LATE genes between cell types using scRNAseq** | | | |
|---|---|---|---|
| **Cell type** | **# shared expressed genes (scRNAseq)** | **# shared LATE genes (scRNAseq)** | **Fraction shared LATE genes (%)** |
| **NK cells, vs Mono.** | 2606 | 9 | 0,35 |
| **NK cells vs B Lymph.** | 2565 | 7 | 0,27 |
| **B Lymph. vs T Lymph.** | 2542 | 6 | 0,24 |
| **NK cells vs T Lymph.** | 2562 | 5 | 0,20 |
| **B Lymph, vs Mono.** | 2613 | 5 | 0,19 |
| **T Lymph. vs Mono** | 2429 | 3 | 0,12 |

### LOY in blood is associated with changes in the plasma proteome

We hypothesized that LOY might leave a footprint in the plasma proteome in subjects with high frequency of leukocytes with this aneuploidy. The ultra-sensitive Proximity Extension Assay (PEA) (Assarsson and Lundberg, 2017) has previously (Enroth et al., 2018) been used in the NSPHS-cohort (Igl et al., 2010) to characterize protein abundance levels in plasma. In brief, abundance levels of 424 plasma proteins have been quantified using five commercially available PEA-panels (Olink Proteomics AB; INF, NEU, ONC2, CVD2 and CVD3), measuring abundance of 92 proteins each. We estimated the LOY status in 480 males in NSPHS from 30X whole genome sequencing data, showing that 13 (2.7%), 36 (7.5%) and 159 (33%) of male NSPHS-subjects had LOY in more than 20%, 10%, and 1.5% of leukocytes, respectively (**Fig. 7A**). By comparing the levels of plasma proteins in men with LOY in more than 20% of leukocytes with men without aneuploidy, using a statistical model adjusted for confounders such as age, smoking, various life style factors and medical history (Enroth et al., 2015), we found many plasma proteins with changed abundance between the groups (**Fig**. **7B**). After strict Bonferroni correction, only one protein remained significant, i.e. chemokine CXCL5, which was three times more abundant in plasma from men with LOY. CXCL5 is a proangiogenic chemokine and a strong attractant for granulocytes and is secreted by various immune- and specific non-immune cells. In the context of cancer, CXCL5 has also been associated with late-stage disease and promotion of metastases (Begley et al., 2008; Hu et al., 2018; Soler-Cardona et al., 2018). Since the CXCL5 gene is not among the LATE genes, caution is advised regarding cause and effect of high CXCL5 plasma levels in subjects with LOY. This might reflect an ongoing cancer-related process in these individuals. Among the other top-hit signals, there was an overrepresentation of proteins involved in regulation of the immune system and the inflammation panel (INF) generated highest number of candidates.

Overall, we did not observe a large overlap between LATE genes detected at the mRNA level within leukocytes and corresponding changes in abundance of their respective plasma proteins (details not shown). However, one of the proteins showing a tendency for higher abundance in men with LOY was TCL1A (**Fig. 7B**). Variants of this gene has been suggested in GWAS (as one of numerous genes) to be associated with increased risk for LOY (Thompson et al., 2019; Wright et al., 2017; Zhou et al., 2016). The present and previous study (Thompson et al., 2019) show that *TCL1A* is predominantly expressed in CD19+ B lymphocytes and overexpressed in cells from this subset affected by LOY, while the other types of leukocytes did not show its expression. However, it is remarkable that increased abundance of the TCL1A protein was detected in plasma from men with LOY. This is because the intracellular location of TCL1A is the cell nucleus (Paduano et al., 2018) and that CD19+ B lymphocytes represent only a few percent of leukocytes in circulation. Results from scRNAseq and protein data, supports that LOY-linked upregulation of *TCL1A* oncogene in B-lymphocytes plays a role in driving clonal expansion in this subset. *TCL1A* (T-Cell Leukemia/Lymphoma 1A) is a gene normally expressed in early stage lymphocytes involved in regulation of multiple signaling pathways. Dysregulation of *TCL1A* has been associated with lymphomagenesis and cancer progression, e.g. in T cell prolymphocytic leukemia, via translocation that brings *TCL1A* under control of *TCR* (Paduano et al., 2018). The observation of *TCL1A* upregulation in B lymphocytes with LOY may suggest a novel LOY-dependent mode of activation for this oncogene, which deserves further studies. Our protein-related results provide a proof of concept that LOY can influence plasma protein levels and suggests that LOY may have an impact on systemic homeostasis. In conclusion, regardless of cause-and-effect relationships, CXCL5 and additional near significance signals showed in **Figure 7** should be studied further using a larger collection of men with high level of LOY in leukocytes.

### LOY has profound effects on transcriptome in pleiotropic fashion

LOY has already been shown as the most common post-zygotic mutation considering data from blood DNA alone (Forsberg et al., 2018; Forsberg et al., 2017). Furthermore, LOY also occurs in other tissues (Forsberg et al., 2018; Haitjema et al., 2017), but these two reports only scratched the surface of the topic and studies of other tissues/cell types should follow in order to establish the frequency of LOY in adult and aging men across the soma. Moreover, as we show here, LOY occurs frequently in blood as oligo-clonal expansions and this could be best explained by independent mutations occurring in progenitors for different lineages of hematopoietic cells, resulting in more than one expanding LOY-clone circulating in blood. Considering the above reasoning, LOY should probably be viewed as the most common human mutation of all categories.

The combined number of LATE genes derived from analyses of various cells is surprisingly large, which suggests that a loss of ~2% of the human genome (via LOY) has a profound impact on cellular homeostasis. Furthermore, we show a limited overlap in the number of LATE genes between different types of hematopoietic cells. We therefore hypothesize that LOY has pleiotropic effects, depending on which cell type (i.e. a distinct normal transcriptomic program) that is disturbed by this large mutation. As already mentioned, epidemiological studies have associated LOY in blood to a number of different diseases and understanding of the underlying mechanisms of LOY in leukocytes on the development of pathologic processes in other tissues is the major future challenge. In this perspective, the LOY-pleiotropy might be a useful concept.

In total, we show 4026 LATE genes with varying degree of differential expression. The analyses of leukocytes via sorted- and single-cells *in vivo* identified 3360 LATE genes. We applied several steps of increasing the stringency for selection of genes with strong statistical support; i.e. robust differences in expression between cells with and without LOY. Table 4 below shows a list of 134 of these LATE genes that were identified as being of interest with respect to continued research into immune-, cancer- and AD-related mechanisms. Many of these genes are involved in normal functions of the immune system. Table 2 above shows a list of 95 genes of particular interest. In our initial papers suggesting the role of LOY in the development of cancer and AD (Dumanski et al., 2016; Forsberg et al., 2014), we proposed that this aneuploidy might impair functions of the immune system, especially immune-surveillance that is responsible for elimination of abnormal cells and structures throughout the soma. In conclusion, the current work provides support for this hypothesis and indicates a role of LOY in distinct populations of leukocytes for the pathogenesis of cancer and Alzheimer's disease.

**Table 4**

| | | | | | | |
|---|---|---|---|---|---|---|
| *A TF3* | *FCER1G* | *GALNT2* | *ISG15* | *MIIP* | *PADI2* | *PYHIN1* |
| *RPS27* | *S100A12* | *SNORA 148* | *SNORA16A* | *SNORA59A* | *SNORA66* | *IFIT1* |
| *NCOA4* | *CADM1* | *CCDC85B* | *CD3D* | *CDKN1C* | *IFITM3* | *SLC22A18* |
| *SNORA23* | *SNORA52* | *SNORA57* | *SNORD 15A* | *SNORD158* | *SORL1* | *ST14* |
| *KLRG1* | *LAG3* | *OAS2* | *OASL* | *SNORA2A* | *SNORA34* | *EPSTI1* |
| *RPS29* | *SNORA28* | *TCL1A* | *TTC9* | *LITAF* | *MT2A* | *PYCARD* |
| *SNORA10* | *SNORA46* | *SNORA50* | *ABI3* | *CCL3* | *CCL5* | *CD7* |
| *SLC9A3R1* | *SNORA21* | *SNORA48* | *SNORA67* | *APBA3* | *CFD* | *FLT3LG* |
| *FXYD5* | *JAK3* | *LILRA1* | *LILRB2* | *PRSS57* | *RPS15* | *RPS5* |
| *SNORA68* | *VASP* | *MARCO* | *MERTK* | *RANBP2* | *SNORA75* | *SNORD94* |
| *ZAP70* | *SNORA71A* | *SNORA71B* | *SNORA71D* | *SNORD17* | *TGIF2* | *MX1* |
| *USP18* | *ALCAM* | *CTNNB1* | *GPX1* | *MRAS* | *PFKFB4* | *RPSA* |
| *SNORA62* | *SNORA63* | *SNORA81* | *TIGIT* | *HERC6* | *IL15* | *PPBP* |
| *RPL34* | *SNORA24* | CD74 | *CLPTM1L* | *CSF1R* | *EGR1* | *GPR150* |
| *PCDHGB5* | *PRLR* | *SNORA47* | *SNORA74A* | *SNORA74B* | *HLA-DPA1* | *HLA-DPB1* |
| *HLA-DQA1* | *HLA-DQB1* | *HLA-DRA* | *HLA-DRB5* | *RLP10A* | *RPS12* | *SNORA38* |
| *LEP* | *PARP12* | *SNORA5A* | *SNORA5C* | *SNORA9* | *ZKSCAN1* | *LY6E* |
| *MYOM2* | *RPL7* | *ANXA1* | *DDX58* | *SNORA65* | *SNORA84* | *USP9Y* |
| *CD99* | *CSF2RA* | *SLC25A6* | *LAGE3* | *P2RY10* | *SLC25A6* | *SNORA70* |
| *CD3G* | | | | | | |

### References

Al Hossiny, M., Luo, L., Frazier, W.R., Steiner, N., Gusev, Y., Kallakury, B., Glasgow, E., Creswell, K., Madhavan, S., Kumar, R., et al. (2016). Ly6E/K Signaling to TGFbeta Promotes Breast Cancer Progression, Immune Escape, and Drug Resistance. Cancer Res 76, 3376-3386.
Araga, S., Kagimoto, H., Funamoto, K., and Takahashi, K. (1991). Reduced natural killer cell activity in patients with dementia of the Alzheimer type. Acta Neurol Scand 84, 259-263.
Assarsson, E., and Lundberg, M. (2017). Development and validation of customized PEA biomarker panels with clinical utility. IN: Advancing precision medicine: Current and future proteogenomic strategies for biomarker discovery and development (Washington, DC: Science/AAAS), pp. 32-36.
Austad, S.N. (2006). Why women live longer than men: sex differences in longevity. Gend Med 3, 79-92.
Begley, L.A., Kasina, S., Mehra, R., Adsule, S., Admon, A.J., Lonigro, R.J., Chinnaiyan, A.M., and Macoska, J.A. (2008). CXCL5 promotes prostate cancer progression. Neoplasia 10, 244-254.
Bellott, D.W., Hughes, J.F., Skaletsky, H., Brown, L.G., Pyntikova, T., Cho, T.J., Koutseva, N., Zaghlul, S., Graves, T., Rock, S., et al. (2014). Mammalian Y chromosomes retain widely expressed dosage-sensitive regulators. Nature 508, 494-499.
Blatt Kalben, B. (2000). Why Men Die Younger. North American Actuarial Journal 4, 83-111.
Butler, A., Hoffman, P., Smibert, P., Papalexi, E., and Satija, R. (2018). Integrating single-cell transcriptomic data across different conditions, technologies, and species. Nat Biotechnol 36, 411-420.
Cook, M.B., McGlynn, K.A., Devesa, S.S., Freedman, N.D., and Anderson, W.F. (2011). Sex disparities in cancer mortality and survival. Cancer epidemiology, biomarkers & prevention 20, 1629-1637.
Cortez, D., Marin, R., Toledo-Flores, D., Froidevaux, L., Liechti, A., Waters, P.D., Grutzner, F., and Kaessmann, H. (2014). Origins and functional evolution of Y chromosomes across mammals. Nature 508, 488-493.
Danielsson, M., Halvardson, J., Davies, H., Torabi Moghadam, B., Mattisson, J., Rychlicka-Buniowska, E., Heintz, J., Lannfelt, L., Giedraitis, V., Ingelsson, M., et al. (2019). Intra-individual changes in the frequency of mosaic loss of chromosome Y over time estimated with a new method. submitted.
Dumanski, J.P., Lambert, J.C., Rasi, C., Giedraitis, V., Davies, H., Grenier-Boley, B., Lindgren, C.M., Campion, D., Dufouil, C., European Alzheimer's Disease Initiative, I., et al. (2016). Mosaic Loss of Chromosome Y in Blood Is Associated with Alzheimer Disease. Am J Hum Genet 98, 1208-1219.
Dumanski, J.P., Rasi, C., Lonn, M., Davies, H., Ingelsson, M., Giedraitis, V., Lannfelt, L., Magnusson, P.K., Lindgren, C.M., Morris, A.P., etal. (2015). Smoking is associated with mosaic loss of chromosome Y. Science 347, 81-83.
Enroth, S., Bosdotter Enroth, S., Johansson, A., and Gyllensten, U. (2015). Effect of genetic and environmental factors on protein biomarkers for common non-communicable disease and use of personally normalized plasma protein profiles (PNPPP). Biomarkers 20, 355-364.
Enroth, S., Maturi, V., Berggrund, M., Enroth, S.B., Moustakas, A., Johansson, A., and Gyllensten, U. (2018). Systemic and specific effects of antihypertensive and lipid-lowering medication on plasma protein biomarkers for cardiovascular diseases. Sci Rep 8, 5531.
Forsberg, L., Halvardson, J., Rychlicka, E., Danielsson , M., Torabi Moghadam, B., Mattisson, J., Rasi, C., Davies, H., Lind, L., Giedraitis, V., et al. (2018). Mosaic loss of chromosome Y (LOY) in leukocytes matters. Nature Genetics.
Forsberg, L.A. (2017). Loss of chromosome Y (LOY) in blood cells is associated with increased risk for disease and mortality in aging men. Hum Genet 136, 657-663.
Forsberg, L.A., Gisselsson, D., and Dumanski, J.P. (2017). Mosaicism in health and disease - clones picking up speed. Nat Rev Genet 18, 128-142.
Forsberg, L.A., Rasi, C., Malmqvist, N., Davies, H., Pasupulati, S., Pakalapati, G., Sandgren, J., de Stahl, T.D., Zaghlool, A., Giedraitis, V., etal. (2014). Mosaic loss of chromosome Y in peripheral blood is associated with shorter survival and higher risk of cancer. Nature Genetics 46, 624-628.
Ganster, C., Kampfe, D., Jung, K., Braulke, F., Shirneshan, K., Machherndl-Spandl, S., Suessner, S., Bramlage, C.P., Legler, T.J., Koziolek, M.J., etal. (2015). New data shed light on Y-loss-related pathogenesis in myelodysplastic syndromes. Genes Chromosomes Cancer 54, 717-724.
Grassmann, F., Kiel, C., den Hollander, A., Weeks, D., Lotery, A., Cipriani, V., Weber, B., and International Age-related Macular Degeneration Genomics Consortium (IAMDGC) (2018). Y chromosome mosaicism is associated with age-related macular degeneration. European Journal of Human Genetics August 2018*.*
Haitjema, S., Kofink, D., van Setten, J., van der Laan, S., Schoneveld, A., Eales, J., Tomaszewski, M., de Jager, S., Pasterkamp, G., Asselbergs, F., et al. (2017). Loss of Y Chromosome in Blood Is Associated with Major Cardiovascular Events during Follow-up in Men after Carotid Endarterectomy. Circulation: Cardiovascular Genetics 10:e001544*.*
Hu, B., Fan, H., Lv, X., Chen, S., and Shao, Z. (2018). Prognostic significance of CXCL5 expression in cancer patients: a meta-analysis. Cancer Cell Int 18, 68.
Huang, C.T., Workman, C.J., Flies, D., Pan, X., Marson, A.L., Zhou, G., Hipkiss, E.L., Ravi, S., Kowalski, J., Levitsky, H.I., et al. (2004). Role of LAG-3 in regulatory T cells. Immunity 21, 503-513.
Igl, W., Johansson, A., and Gyllensten, U. (2010). The Northern Swedish Population Health Study (NSPHS)--a paradigmatic study in a rural population combining community health and basic research. Rural Remote Health 10, 1363.
Jacobs, P.A., Brunton, M., Court Brown, W.M., Doll, R., and Goldstein, H. (1963). Change of human chromosome count distribution with age: evidence for a sex differences. Nature 197, 1080-1081.
Jadidi-Niaragh, F., Shegarfi, H., Naddafi, F., and Mirshafiey, A. (2012). The role of natural killer cells in Alzheimer's disease. Scand J Immunol 76, 451-456.
Korin, B., Ben-Shaanan, T.L., Schiller, M., Dubovik, T., Azulay-Debby, H., Boshnak, N.T., Koren, T., and Rolls, A. (2017). High-dimensional, single-cell characterization of the brain's immune compartment. Nat Neurosci 20, 1300-1309.
Larbi, A., Pawelec, G., Witkowski, J.M., Schipper, H.M., Derhovanessian, E., Goldeck, D., and Fulop, T. (2009). Dramatic shifts in circulating CD4 but not CD8 T cell subsets in mild Alzheimer's disease. J Alzheimers Dis 17, 91-103.
Le Page, A., Bourgade, K., Lamoureux, J., Frost, E., Pawelec, G., Larbi, A., Witkowski, J.M., Dupuis, G., and Fulop, T. (2015). NK Cells are Activated in Amnestic Mild Cognitive Impairment but not in Mild Alzheimer's Disease Patients. J Alzheimers Dis 46, 93-107.
Le Page, A., Dupuis, G., Frost, E.H., Larbi, A., Pawelec, G., Witkowski, J.M., and Fulop, T. (2018). Role of the peripheral innate immune system in the development of Alzheimer's disease. Exp Gerontol 107, 59-66.
Lleo, A., Oertelt-Prigione, S., Bianchi, I., Caliari, L., Finelli, P., Miozzo, M., Lazzari, R., Floreani, A., Donato, F., Colombo, M., et al. (2013). Y chromosome loss in male patients with primary biliary cirrhosis. Journal of autoimmunity 41, 87-91.
Loftfield, E., Zhou, W., Graubard, B.I., Yeager, M., Chanock, S.J., Freedman, N.D., and Machiela, M.J. (2018). Predictors of mosaic chromosome Y loss and associations with mortality in the UK Biobank. Sci Rep 8, 12316.
Loftfield, E., Zhou, W., Yeager, M., Chanock, S.J., Freedman, N.D., and Machiela, M.J. (2019). Mosaic Y Loss Is Moderately Associated with Solid Tumor Risk. Cancer Res 79, 461-466.
Love, M.I., Huber, W., and Anders, S. (2014). Moderated estimation of fold change and dispersion for RNA-seq data with DESeq2. Genome Biol 15, 550.
Luo, L., McGarvey, P., Madhavan, S., Kumar, R., Gusev, Y., and Upadhyay, G. (2016). Distinct lymphocyte antigens 6 (Ly6) family members Ly6D, Ly6E, Ly6K and Ly6H drive tumorigenesis and clinical outcome. Oncotarget 7, 11165-11193.
Masera, R.G., Prolo, P., Sartori, M.L., Staurenghi, A., Griot, G., Ravizza, L., Dovio, A., Chiappelli, F., and Angeli, A. (2002). Mental deterioration correlates with response of natural killer (NK) cell activity to physiological modifiers in patients with short history of Alzheimer's disease. Psychoneuroendocrinology 27, 447-461.
Mrdjen, D., Pavlovic, A., Hartmann, F.J., Schreiner, B., Utz, S.G., Leung, B.P., Lelios, I., Heppner, F.L., Kipnis, J., Merkler, D., etal. (2018). High-Dimensional Single-Cell Mapping of Central Nervous System Immune Cells Reveals Distinct Myeloid Subsets in Health, Aging, and Disease. Immunity 48, 380-395 e386.
Noveski, P., Madjunkova, S., Sukarova Stefanovska, E., Matevska Geshkovska, N., Kuzmanovska, M., Dimovski, A., and Plaseska-Karanfilska, D. (2016). Loss of Y Chromosome in Peripheral Blood of Colorectal and Prostate Cancer Patients. PLoS One 11, e0146264.
Nowinski, G.P., Van Dyke, D.L., Tilley, B.C., Jacobsen, G., Babu, V.R., Worsham, M.J., Wilson, G.N., and Weiss, L. (1990). The frequency of aneuploidy in cultured lymphocytes is correlated with age and gender but not with reproductive history. Am J Hum Genet 46, 1101-1111.
Paaby, A.B., and Rockman, M.V. (2013). The many faces of pleiotropy. Trends Genet 29, 66-73.
Paduano, F., Gaudio, E., Mensah, A.A., Pinton, S., Bertoni, F., and Trapasso, F. (2018). T-Cell Leukemia/Lymphoma 1 (TCL1): An Oncogene Regulating Multiple Signaling Pathways. Front Oncol 8, 317.
Persani, L., Bonomi, M., Lleo, A., Pasini, S., Civardi, F., Bianchi, I., Campi, I., Finelli, P., Miozzo, M., Castronovo, C., et al. (2012). Increased loss of the Y chromosome in peripheral blood cells in male patients with autoimmune thyroiditis. Journal of autoimmunity 38, J193-196.
Pierre, R.V., and Hoagland, H.C. (1972). Age-associated aneuploidy: loss of Y chromosome from human bone marrow cells with aging. Cancer 30, 889-894.
Schenkel, A.R., Mamdouh, Z., Chen, X., Liebman, R.M., and Muller, W.A. (2002). CD99 plays a major role in the migration of monocytes through endothelial junctions. Nat Immunol 3, 143-150.
Schindowski, K., Peters, J., Gorriz, C., Schramm, U., Weinandi, T., Leutner, S., Maurer, K., Frolich, L., Muller, W.E., and Eckert, A. (2006). Apoptosis of CD4+ T and natural killer cells in Alzheimer's disease. Pharmacopsychiatry 39, 220-228.
Soler-Cardona, A., Forsthuber, A., Lipp, K., Ebersberger, S., Heinz, M., Schossleitner, K., Buchberger, E., Groger, M., Petzelbauer, P., Hoeller, C., et al. (2018). CXCL5 Facilitates Melanoma Cell-Neutrophil Interaction and Lymph Node Metastasis. J Invest Dermatol 138, 1627-1635.
Solerte, S.B., Cravello, L., Ferrari, E., and Fioravanti, M. (2000). Overproduction of IFN-gamma and TNF-alpha from natural killer (NK) cells is associated with abnormal NK reactivity and cognitive derangement in Alzheimer's disease. Ann N Y Acad Sci 917, 331-340.
Speciale, L., Calabrese, E., Saresella, M., Tinelli, C., Mariani, C., Sanvito, L., Longhi, R., and Ferrante, P. (2007). Lymphocyte subset patterns and cytokine production in Alzheimer's disease patients. Neurobiol Aging 28, 1163-1169.
Sullivan, K.M., Mannucci, A., Kimpton, C.P., and Gill, P. (1993). A rapid and quantitative DNA sex test: fluorescence-based PCR analysis of X-Y homologous gene amelogenin. Biotechniques 15, 636-638, 640-631.
Thompson, D., Genovese, G., Halvardson, J., Ulirsch, J., Wright, D., Terao, C., Davidsson, O., Day, F., Sulem, P., Jiang, Y., et al. (2019). Genetic predisposition to mosaic Y chromosome loss in blood is associated with genomic instability in other tissues and susceptibility to non-haematological cancers. submitted.
UKCCG (1992). Loss of the Y chromosome from normal and neoplastic bone marrows. United Kingdom Cancer Cytogenetics Group (UKCCG). Genes, chromosomes cancer 5, 83-88.
Vestweber, D. (2015). How leukocytes cross the vascular endothelium. Nat Rev Immunol 15, 692-704.
Wiktor, A., Rybicki, B.A., Piao, Z.S., Shurafa, M., Barthel, B., Maeda, K., and Van Dyke, D.L. (2000). Clinical significance of Y chromosome loss in hematologic disease. Genes, chromosomes & cancer 27, 11-16.
Workman, C.J., Cauley, L.S., Kim, I.J., Blackman, M.A., Woodland, D.L., and Vignali, D.A. (2004). Lymphocyte activation gene-3 (CD223) regulates the size of the expanding T cell population following antigen activation in vivo. J Immunol 172, 5450-5455.
Wright, D.J., Day, F.R., Kerrison, N.D., Zink, F., Cardona, A., Sulem, P., Thompson, D.J., Sigurjonsdottir, S., Gudbjartsson, D.F., Helgason, A., etal. (2017). Genetic variants associated with mosaic Y chromosome loss highlight cell cycle genes and overlap with cancer susceptibility. Nat Genet 49, 674-679.
Zhou, W., Machiela, M.J., Freedman, N.D., Rothman, N., Malats, N., Dagnall, C., Caporaso, N., Teras, L.T., Gaudet, M.M., Gapstur, S.M., et al. (2016). Mosaic loss of chromosome Y is associated with common variation near TCL1A. Nat Genet 48, 563-568.
Zink, F., Stacey, S.N., Norddahl, G.L., Frigge, M.L., Magnusson, O.T., Jonsdottir, I., Thorgeirsson, T.E., Sigurdsson, A., Gudjonsson, S.A., Gudmundsson, J., etal. (2017). Clonal hematopoiesis, with and without candidate driver mutations, is common in the elderly. Blood 130, 742-752.

## Claims

1. A method of stratifying a male subject for immunotherapy, said method comprising:
(a) determining in a sample obtained from the male subject a fraction of leucocytes that have lost chromosome Y;
(b) comparing said fraction to a predefined threshold; and
(c) based on said comparison between said fraction and said predefined threshold, assigning the male subject to either a LOY+ or LOY- sub-group, and stratifying the subject for said immunotherapy based on said sub-group.

2. A method of identifying the likelihood of response of a male subject to an immunotherapy, said method comprising:
(a) determining in a sample from the subject the fraction of leucocytes that have lost chromosome Y; and
(b) correlating the fraction of leucocytes that have lost chromosome Y to the likelihood that the subject will respond to treatment with the immunotherapy.

3. The method of claim 2, wherein step (b) comprises comparing the fraction of leucocytes that have lost chromosome Y to a predefined threshold.

4. The method of any one of claims 1 to 3, wherein the leucocytes are selected from the group consisting of T-cells, B-cells, NK cells, monocytes, or granulocytes, or a combination thereof.

5. The method of claim 4, wherein the leucocytes are CD4+ T-cells or CD8+ T-cells.

6. The method of claim 4, wherein the leucocytes are NK cells.

7. The method of any one of claims 1 to 6, further comprising determining the level of expression of one of more biomarkers in a sample from the subject, wherein the biomarkers are selected from the biomarkers listed in Table 4 or Table 2.

8. The method of claim 7, wherein the subject is assigned for said immunotherapy based on the comparison of the level of expression to the predefined threshold.

9. The method of any one of claims 7 or 8, wherein the biomarker is one of more of LAG3, LY6E, ANXA1, CSF1R, APBA3, CSF2RA, EGR1, JAK3, ISG15, IL15, CD3D, CD3G, CADM1, PYCARD, USP18, USP9Y, LILRB2, LILRA1, VASP, ZAP70, KLRG1, P2RY10, CFD, CCL3, FCERG, HLA-DRA and CCL5.

10. The method of any one of claims 7 to 9, wherein the biomarker is LAG3 and/or LYE6.

11. The method of any one of claims 1 to 10, wherein the sample comprises leucocytes, or a subset thereof.

12. The method of any one of claims 1 to 11, said method further comprising recommending or prescribing an immunotherapy to the subject.

13. The method of any one of claims 1 to 12, wherein the immunotherapy comprises an immune checkpoint inhibitor.

14. The method of claim 13, wherein:
(i) the immunotherapy comprises an immune checkpoint inhibitor which inhibits an immune checkpoint selected from the group consisting of LAG-3, KIR, CD244, ICOS, CD160, CD47, and TIGIT; and/or
(ii) the immunotherapy comprises an antibody; and/or
(iii) the immunotherapy comprises an immune checkpoint inhibitor which inhibits LAG-3.

15. The method of any one of claims 1 to 14, wherein for determining LOY said predefined threshold is selected from 5, 7, 8, 10, 13, 18, 20, 25, 30 or 40% of cells in the sample that have lost chromosome Y.

## Patentansprüche

1. Verfahren zum Stratifizieren eines männlichen Patienten für eine Immuntherapie, wobei das Verfahren Folgendes umfasst:
(a) Bestimmen einer Fraktion von Leukozyten, die das Y-Chromosom verloren haben, in einer von dem männlichen Patienten erhaltenen Probe;
(b) Vergleichen der Fraktion mit einem vordefinierten Grenzwert; und
(c) basierend auf dem Vergleich zwischen der Fraktion und dem vordefinierten Grenzwert, Zuordnen des männlichen Patienten entweder einer LOY+ oder LOY-Subgruppe und Stratifizieren des Patienten für die Immuntherapie, basierend auf der Subgruppe.

2. Verfahren zum Identifizieren der Wahrscheinlichkeit einer Reaktion eines männlichen Patienten auf eine Immuntherapie, wobei das Verfahren Folgendes umfasst:
(a) Bestimmen der Fraktion von Leukozyten, die das Y-Chromosom verloren haben, in einer Probe von dem Patienten; und
(b) Korrelieren der Fraktion von Leukozyten, die das Y-Chromosom verloren haben, mit der Wahrscheinlichkeit, dass der Patient auf eine Behandlung mit der Immuntherapie ansprechen wird.

3. Verfahren nach Anspruch 2, wobei Schritt (b) das Vergleichen der Fraktion von Leukozyten, die das Y-Chromosom verloren haben, mit einem vordefinierten Grenzwert umfasst.

4. Verfahren nach einem der Ansprüche 1 bis 3, wobei die Leukozyten ausgewählt sind aus der Gruppe bestehend aus T-Zellen, B-Zellen, NK-Zellen, Monozyten oder Granulozyten oder einer Kombination davon.

5. Verfahren nach Anspruch 4, wobei die Leukozyten CD4+ T-Zellen oder CD8+ T-Zellen sind.

6. Verfahren nach Anspruch 4, wobei die Leukozyten NK-Zellen sind.

7. Verfahren nach einem der Ansprüche 1 bis 6, weiter umfassend das Bestimmen des Expressionswertes eines von mehreren Biomarkern in einer Probe von dem Patienten, wobei die Biomarker ausgewählt sind aus den in Tabelle 4 oder Tabelle 2 aufgeführten Biomarkern.

8. Verfahren nach Anspruch 7, wobei der Patient der Immuntherapie basierend auf dem Vergleich des Expressionswertes mit dem vordefinierten Grenzwert zugeordnet wird.

9. Verfahren nach einem der Ansprüche 7 oder 8, wobei der Biomarker einer oder mehrere von LAG3, LY6E, ANXA1, CSF1R, APBA3, CSF2RA, EGR1, JAK3, ISG15, IL15, CD3D, CD3G, CADM1, PYCARD, USP18, USP9Y, LILRB2, LILRA1, VASP, ZAP70, KLRG1, P2RY10, CFD, CCL3, FCERG, HLA-DRA und CCL5 ist.

10. Verfahren nach einem der Ansprüche 7 bis 9, wobei der Biomarker LAG3 und/oder LYE6 ist.

11. Verfahren nach einem der Ansprüche 1 bis 10, wobei die Probe Leukozyten oder eine Subsatz davon umfasst.

12. Verfahren nach einem der Ansprüche 1 bis 11, wobei das Verfahren weiter eine Empfehlung oder Verschreibung einer Immuntherapie für den Patienten umfasst.

13. Verfahren nach einem der Ansprüche 1 bis 12, wobei die Immuntherapie einen Immuncheckpoint-Inhibitor umfasst.

14. Verfahren nach Anspruch 13, wobei:
(i) die Immuntherapie einen Immuncheckpoint-Inhibitor umfasst, der einen Immuncheckpoint hemmt, der ausgewählt ist aus der Gruppe bestehend aus LAG-3, KIR, CD244, ICOS, CD160, CD47 und TIGIT; und/oder
(ii) die Immuntherapie einen Antikörper umfasst; und/oder
(iii) die Immuntherapie einen Immuncheckpoint-Inhibitor umfasst, der LAG-3 hemmt.

15. Verfahren nach einem der Ansprüche 1 bis 14, wobei der vordefinierte Grenzwert zum Bestimmen von LOY ausgewählt ist aus 5, 7, 8, 10, 13, 18, 20, 25, 30 oder 40 % von Zellen in der Probe, die das Y-Chromosom verloren haben.

## Revendications

1. Procédé de stratification d'un sujet mâle pour une immunothérapie, ledit procédé comprenant les étapes consistant à :
(a) déterminer, dans un échantillon prélevé sur le sujet mâle, une fraction de leucocytes ayant perdu le chromosome Y ;
(b) comparer ladite fraction à un seuil prédéfini ; et
(c) sur la base de ladite comparaison entre ladite fraction et ledit seuil prédéfini, affecter le sujet mâle à un sous-groupe LOY+ ou LOY-, et stratifier le sujet pour ladite immunothérapie sur la base dudit sous-groupe.

2. Procédé d'identification de la probabilité de réponse d'un sujet mâle à une immunothérapie, ledit procédé comprenant les étapes consistant à :
(a) déterminer, dans un échantillon provenant du sujet, la fraction de leucocytes ayant perdu le chromosome Y ; et
(b) corréler la fraction de leucocytes ayant perdu le chromosome Y à la probabilité que le sujet réponde au traitement par immunothérapie.

3. Procédé selon la revendication 2, dans lequel l'étape (b) comprend la comparaison de la fraction de leucocytes ayant perdu le chromosome Y à un seuil prédéfini.

4. Procédé selon l'une quelconque des revendications 1 à 3, dans lequel les leucocytes sont sélectionnés parmi le groupe consistant en les cellules T, les cellules B, les cellules NK, les monocytes, ou les granulocytes, ou une combinaison de ceux-ci.

5. Procédé selon la revendication 4, dans lequel les leucocytes sont des cellules T CD4+ ou des cellules T CD8+.

6. Procédé selon la revendication 4, dans lequel les leucocytes sont des cellules NK.

7. Procédé selon l'une quelconque des revendications 1 à 6, comprenant en outre la détermination du niveau d'expression d'un ou de plusieurs biomarqueurs dans un échantillon provenant du sujet, dans lequel les biomarqueurs sont sélectionnés parmi les biomarqueurs listés dans le tableau 4 ou le tableau 2.

8. Procédé selon la revendication 7, dans lequel le sujet est affecté à ladite immunothérapie sur la base de la comparaison du niveau d'expression au seuil prédéfini.

9. Procédé selon l'une quelconque des revendications 7 ou 8, dans lequel le biomarqueur est un ou plusieurs parmi LAG3, LY6E, ANXA1, CSF1R, APBA3, CSF2RA, EGR1, JAK3, ISG15, IL15, CD3D, CD3G, CADM1, PYCARD, USP18, USP9Y, LILRB2, LILRA1, VASP, ZAP70, KLRG1, P2RY10, CFD, CCL3, FCERG, HLA-DRA et CCL5.

10. Procédé selon l'une quelconque des revendications 7 à 9, dans lequel le biomarqueur est LAG3 et/ou LYE6.

11. Procédé selon l'une quelconque des revendications 1 à 10, dans lequel l'échantillon comprend des leucocytes, ou un sous-ensemble de ceux-ci.

12. Procédé selon l'une quelconque des revendications 1 à 11, ledit procédé comprenant en outre la recommandation ou la prescription d'une immunothérapie au sujet.

13. Procédé selon l'une quelconque des revendications 1 à 12, dans lequel l'immunothérapie comprend un inhibiteur de point de contrôle immunitaire.

14. Procédé selon la revendication 13, dans lequel :
(i) l'immunothérapie comprend un inhibiteur de point de contrôle immunitaire qui inhibe un point de contrôle immunitaire sélectionné parmi le groupe consistant en LAG-3, KIR, CD244, ICOS, CD160, CD47, et TIGIT ; et/ou
(ii) l'immunothérapie comprend un anticorps ; et/ou
(iii) l'immunothérapie comprend un inhibiteur de point de contrôle immunitaire qui inhibe LAG-3.

15. Procédé selon l'une quelconque des revendications 1 à 14, dans lequel, pour déterminer le LOY, ledit seuil prédéfini est sélectionné parmi 5, 7, 8, 10, 13, 18, 20, 25, 30 ou 40 % des cellules de l'échantillon ayant perdu le chromosome Y.
